(19) Europäisches Patentamt
European Patent Office
Office européen des brevets

(11) EP 4 389 148 A2

(12) EUROPEAN PATENT APPLICATION

(43) Date of publication:
**26.06.2024 Bulletin 2024/26**

(21) Application number: **24174393.9**

(22) Date of filing: **19.06.2020**

(51) International Patent Classification (IPC):
***A61K 47/12*** *(2006.01)*

(52) Cooperative Patent Classification (CPC):
**A61K 9/501; A61K 33/00; A61K 47/06;**
A61K 9/0014; A61K 9/0019; Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2019 US 201962863503 P**

(62) Document number(s) of the earlier application(s) in accordance with Art. 76 EPC:
**20827734.3 / 4 048 289**

(71) Applicant: **Zylo Therapeutics, Inc.**
**Greenville, SC 29615 (US)**

(72) Inventor: **DRAGANSKI, Andrew**
**Greenville (US)**

(74) Representative: **Dummett Copp LLP**
**25 The Square**
**Martlesham Heath**
**Ipswich IP5 3SL (GB)**

Remarks:
This application was filed on 06-05-2024 as a divisional application to the application mentioned under INID code 62.

# (54) SULFUR FUNCTIONALIZED MONOLITHS AND PARTICLES DERIVED FROM THE SAME AS NITRIC OXIDE CARRIERS FOR PHARMACEUTICAL AND COSMETIC APPLICATIONS

(57) The present application is directed to sulfur-functionalized monoliths and particles derived therefrom as nitric oxide (NO) carriers for applications in drug delivery and cosmetic formulations. An example aspect of the application includes a monolith structure which contains a first monomer having a sulfur functionalized side group linked with a second monomer to form a framework. Another aspect of the application includes a particle having a rough surface derived from the monolith. A further aspect of the application includes the particle having a rough surface which has been reacted to form a nitrosylated particle containing NO covalently attached to a sulfur group. As also described in the application, aspects of the disclosure can include methods for making the monolith structure, methods for deriving a particle or particle having a rough surface from the monolith, and methods for treating a condition using a nitrosylated particle or a particle loaded with NO.

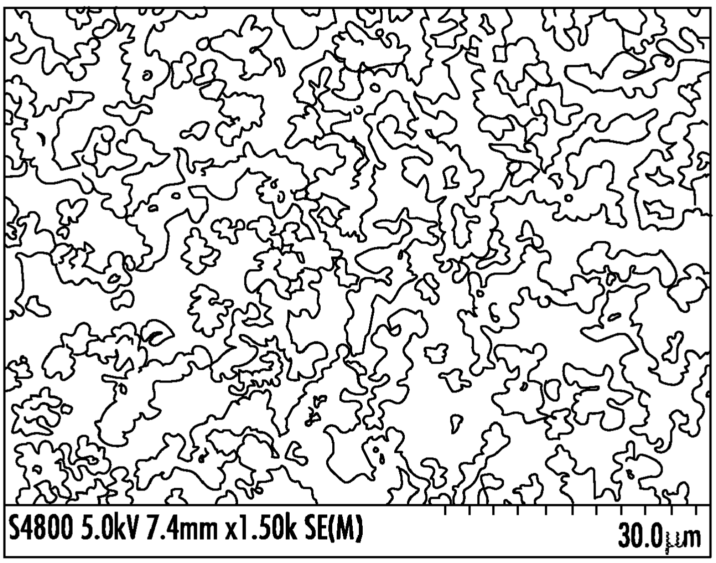


FIG. 2A

EP 4 389 148 A2

## Description

CROSS REFERENCE TO RELATED APPLICATIONS

The present application claims priority to United States Provisional Application No. 62/863,503, having a filing date of June 19, 2019, the disclosure of which is incorporated herein by reference in its entirety.

BACKGROUND

Ceramic particles such as silica, alumina, and aluminum silicates are promising drug carriers because ceramics possess favorable chemical properties, thermal stability, and biocompatibility. Additionally, the production of certain ceramics can be accomplished by a variety of synthesis routes using different monomers to modify the properties for certain applications. One method of producing silica particles is the Stöber method - a nucleation/ growth sol-gel process - in which a hydrolysable silane precursor is reacted with water in an alcohol solution. However, there are several drawbacks to this process as the conditions can require low concentration of alkoxysilane monomer, extended reaction times under constant shear with steady and continual introduction of monomer, and incomplete conversion of monomer to particles. The result of the Stöber process are small and monodisperse particles, which may be useful for certain applications; however, the process has several disadvantages for large scale manufacture due in part to long reaction times, low yields, and expense.

Following discovery of the Stöber process, a two-stage modification was reported that allowed the patterned formation of pores in the silica particles to produce mesoporous silica particles. Due to the large surface area of the pores, these particles have great potential for applications in drug delivery. The fabrication involves reacting a silane precursor with a self-assembled hexagonal arrangement of cylindrical mesopores. Still, this new process has drawbacks including low yield and the need to remove surfactant to assure biocompatibility.

The application of silica particles as delivery agents for drugs (e.g., proteins) by conjugation with surface reactive groups or by loading into pores have provided new routes for drug delivery that have improved disease targeting, reduced side effects, and improved patient outcomes. However, there is still room for significant advancement to improve manufacturing processes and thus reduce costs for patients and companies. Additionally, the development of carriers for delivery of certain fugitive small molecules may expand the applications and treatments for which particle carriers can be used.

For example, over the past few decades several nitric oxide (NO)-related therapeutics have emerged. In general, these systems use enzymatic activity to generate NO from a pro-drug. These pro-drugs include organonitrates, most notably nitroglycerine and organometallic NO-donors such as sodium nitroprusside. Disadvantages, such as progressive tachyphylaxis resulting from depletion of host enzymes required for the generation of NO, potential toxicity from toxic byproducts (e.g., sodium nitroprusside decomposes releasing NO as well as cyanide), and short-lived biological impact, all limit their therapeutic efficacy. While gaseous NO is effective and FDA-approved for treatment of pulmonary hypertension, use in patients is limited due to expense, requirement of delivery via gas tank, and potential toxicity issues from the production of $NO_2$.

Since NO has a variety of biological applications, including wound healing and disinfection, needed in the art are methods and systems capable of the spontaneous release of NO that do not require enzymatic release or expensive high-pressure storage tanks.

SUMMARY

The present application is directed to sulfur-functionalized monoliths and particles derived therefrom as nitric oxide (NO) carriers for applications in drug delivery and cosmetic formulations. An example aspect of the application includes a monolith structure which contains a first monomer having a sulfur functionalized side group linked with a second monomer to form a framework. Another aspect of the application includes a particle having a rough surface derived from the monolith. A further aspect of the application includes the particle having a rough surface which has been reacted to form a nitrosylated (e.g., an S-nitroso functionalized) particle containing NO covalently attached to one or more sulfur groups. As also described in the application, aspects of the disclosure can include methods for making the monolith structure, methods for deriving a particle or particle having a rough surface from the monolith, and methods for treating a condition using a nitrosylated particle or a particle loaded with NO. In general, embodiments of the disclosure may provide advantages to other NO delivery systems and methods of manufacture since they can be produced at lower costs and demonstrate bio-compatibility. Additionally, the rough surface of the silica particles provides an unexpected advantage by embedding the carrier particles into the skin, which can improve NO delivery in applications such as topical administration through increased surface contact.

BRIEF DESCRIPTION OF THE DRAWINGS

A full and enabling disclosure of the present invention, including the best mode thereof to one skilled in the art, is set forth more particularly in the remainder of the specification, which includes reference to the accompanying figures.

FIGs. 1A-1H illustrate images of monolith formation after a reaction time in accordance with example embodiments of the disclosure.
FIGs. 2A-2C illustrate images of particles formed in accordance with example embodiments of the disclosure at varying magnification.
FIG. 3 illustrates a histogram for particle diameters formed in accordance with example embodiments of the disclosure.
FIGs. 4A and 4B illustrate graphs displaying instantaneous NO release and cumulative NO release, respectively, in accordance with example embodiments of the disclosure.
FIGs. 5A-5J illustrate images of monolith formation after a reaction time in accordance with example embodiments of the disclosure.
FIG. 6A illustrates a bar graph showing yield vs. condensation time for example embodiments produced at RT or at 40 °C.
FIG. 6B illustrates a bar graph showing mean particle size vs. condensation time for example embodiments formed at RT or at 40 °C.
FIG. 6C illustrates a bar graph showing NO load vs. condensation time for example embodiments formed at RT or at 40 °C.
FIGs. 7A-7C illustrate images of skin tissue in grayscale upon which an example embodiment of the disclosure has been applied.
FIGs. 7D-7F illustrate the images of FIGs. 7A-7C, respectively, in color.
FIGs. 8A-8C illustrate images of cross-sections taken from tissue shown in FIGs. 7A-7C, respectively.
FIGs. 8D-8F illustrate the images of FIGs. 8A-8C, respectively, in color.
FIG. 9 illustrates a graph displaying fluorescence intensity vs. strip number for untreated skin control, 1-hour post application, and 6-hour post application.
FIG. 10 illustrates a graph displaying relative pressure vs. volume.
FIG. 11 illustrates a table displaying data calculated from a BET analysis of an example embodiment formed in accordance with the disclosure.
FIG. 12 illustrates a table displaying data determined from example embodiments formed using the MPTS/TEOS ratio shown in accordance with the disclosure.
FIGs. 13A and 13B illustrate graphs displaying NO ppm and NO release rate, respectively, versus time in accordance with an example embodiment of the disclosure.
FIGs. 14A and 14B illustrate graphs displaying NO ppm and NO release rate, respectively, versus time in accordance with an example embodiment of the disclosure.
FIGs. 15A and 15B illustrate graphs displaying NO release rate and cumulative NO release, respectively, versus time in accordance with an example embodiment of the disclosure.
FIG. 16 illustrates a graph displaying cumulative NO release in accordance with an example embodiment of the disclosure.
FIG. 17 is an illustration showing the particles in accordance with the present disclosure collecting at an opening to a hair follicle of the skin.

Repeat use of reference characters in the present specification and drawings is intended to represent the same or analogous features or elements of the present invention.

DETAILED DESCRIPTION

Reference now will be made to the embodiments of the invention, one or more examples of which are set forth below. Each example is provided by way of an explanation of the invention, not as a limitation of the invention. In fact, it will be apparent to those skilled in the art that various modifications and variations can be made in the invention without departing from the scope or spirit of the invention. For instance, features illustrated or described as one embodiment can be used on another embodiment to yield still a further embodiment. Thus, it is intended that the present invention cover such modifications and variations as come within the scope of the appended claims and their equivalents. It is to be understood by one of ordinary skill in the art that the present discussion is a description of exemplary embodiments only and is not intended as limiting the broader aspects of the present invention, which broader aspects are embodied exemplary constructions.

Generally speaking, the present disclosure is directed to sulfur functionalized monoliths and particles derived from the same as carriers for pharmaceutical and cosmetic applications. The disclosed materials, compositions and methods may provide advantages as their formation occurs quickly over a range of conditions, allowing for fast, efficient, and productive manufacture that can significantly reduce costs for both manufacturers and consumers. Example embodiments of the disclosure can include: a monolith formed from at least two monomers and including a sulfur functional group (e.g., a thiol), particles derived from the monolith, methods of producing the monolith, and methods for forming particles from the monolith. Embodiments of the disclosure can also include compositions that include particles derived from the monolith and treatments using the compositions.

For embodiments of the disclosure, the particles derived from the monolith can be loaded with a compound or a precursor (the precursor, upon exposure to a condition, converting to the compound). As an example, an embodiment of the disclosure can include a cosmetic composition containing particles derived from the monolith. In this example, the particles contained in the cosmetic composition can be loaded with a high vapor pressure compound that under ambient conditions normally exists as a gas (e.g., NO). Nitric oxide is produced by the body and is known to have several biological effects including increasing circulation, which can improve wound healing and may also help decrease the effects of aging, such as wrinkles. Thus, application of the composition to an area on the body can provide a means for localized delivery of the compound.

This example illustrates another advantage that can be realized in practicing embodiments of the disclosure. In certain embodiments, the particles derived from the monolith can have an irregular or rough surface The rough surface can provide multiple advantages, such as increasing the surface area for binding the compound or by imbedding into an application area (e.g., a top layer of skin or mucus membrane). By increasing the surface area, embodiments of the disclosure can provide improved loadings of compound or precursor, which can reduce the frequency of application. By embedding into an outer surface, these embodiments can improve contact time without requiring the presence of a continuous applicator, such as a patch. Since it can be extremely difficult to deliver a fugitive compound, such as a high vapor pressure diatomic molecule, to a localized area, embodiments of the disclosure may provide additional advantages for delivering compounds such as NO.

**[0014]** The particles of the present disclosure are formulated and constructed so to embed into an outer surface, such as the skin of a user. For example, the particles are formed with high surface roughness that not only facilitates embedding of the particles into skin, but also with a particle size that optimized contact with the skin. In one aspect, the particles have a size and shape that causes the particles to congregate at hair follicles on the skin. The hair follicles can act as a funnel for the particles. Once in the hair follicle, the particles not only are protected from removal but are positioned at a location that maximizes release of the compound or precursor, such as nitric oxide. For example, referring to FIG. 17, human skin (ex vivo human tissue) is shown in conjunction with the particles of the present disclosure. As illustrated in the figure, the particles collect in the opening of a hair follicle for maximizing delivery.

An embodiment of the disclosure can include a monolith, the monolith including a first monomer and a second monomer linked to form a framework, the first monomer including a sulfur functionalized side group. Another embodiment of the disclosure can include a particle derived from the monolith. Generally, the particle derived from the monolith is understood to include the same chemical composition as the monolith from which it was derived, unless it has undergone further reaction or processing. Thus, the particle derived from the monolith also includes a first monomer including a sulfur functionalized side group and a second monomer.

In certain embodiments, the first monomer can be present at a molar ratio of about 1:5 to about 5:1 with respect to the second monomer, such as about 1:3 to about 3:1, and about 4:6 to about 6:4. While reference here is only made to the first monomer, it should be understood that the remainder need not only include the second monomer. In some embodiments, a third monomer can be present as part of the monolith or the particle derived from the monolith. In some embodiments, a third monomer and a fourth monomer can be present, such that the monolith or the particle derived from the monolith includes at least 4 different monomers. As such, embodiments of the disclosure are not constrained to only using a first monomer and a second monomer.

Generally, the first monomer may include structures derived from Structure I or Structure II:

Structure I

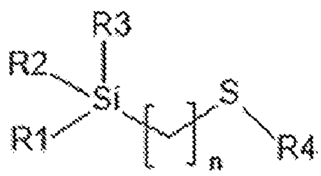

or

Structure II

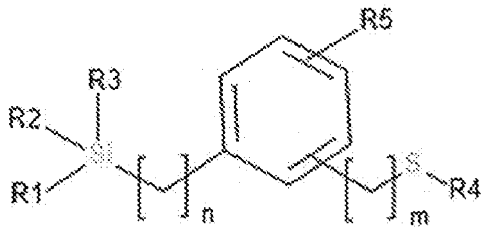

each of R1-R3 is selected independently from, a hydrogen (-H); a hydroxyl (-OH); a halogen (-X) including: fluorine (-F), chlorine (-Cl), or bromine (-Br), or iodine (-I); a linear or branched alkyl group having 1-5 carbon atoms, and an alkoxy group ($-OC_xH_y$) having 1-5 linear or branched carbon atoms. R4 is selected from the group: hydrogen (-H), a linear or branched alkyl group having 1-4 carbon atoms; R5 is selected from the group consisting of: hydrogen, a linear or branched alkyl group having 1-3 carbon atoms, a hydroxyl (-OH), a thiol (-SH), and a halogen (-X) including fluorine (-F), chlorine (-Cl), and bromine (-Br); and n and m are independently = 1-4. Substituents that are not attached to a carbon atom on the phenyl ring (e.g., R5) may be substituted at any position on the phenyl ring.

Example compounds derived from Structure I include: 3-mercaptoproplytrimethoxysilane (MPTS), 3-mercaptopropyltriethoxysilane (MPTES), and 3-mercaptopropylmethyldimethoxysilane (MPDMS). These examples are provided for illustration only and are not intended to limit the scope of the first monomer only to these compounds. Additionally, combinations of these compounds or other structures derived from Structure I may be included in the monolith or particles derived from the monolith without departing from the spirit and scope of the disclosure.

Generally, the second monomer may include structures derived from Structure III:

Structure III

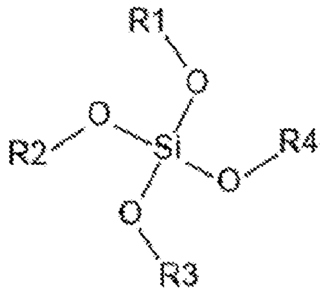

Example compounds derived from Structure III include: tetraethyl orthosilicate (TEOS), tetramethyl orthosilicate (TMOS), tetrapropyl orthosilicate (TPOS), & tetrabutyl orthosilicate (TBOS). These examples are provided for illustration only and are not intended to limit the scope of the second monomer only to these compounds. Additionally, combinations of these compounds or other structures derived from Structure III may be included in the monolith or particles derived from the monolith without departing from the spirit and scope of the disclosure.

While Structure III illustrates a silicate, it should be understood that other metal alkoxides, metal oxides, and salts or chelates thereof can be used in embodiments of the disclosure. For example, in some embodiments, the second monomer can include an alkoxide, a salt, or a chelate of a metal oxide, the metal included in the group: silicon, aluminum, boron, germanium, barium, lithium, sodium, titanium, zirconium, magnesium, strontium, hafnium, and vanadium.

An aspect of certain embodiments can include a third monomer linked to the first monomer and the second monomer as part of the monolith structure or as part of a particle derived from such monolith, the third monomer containing an ionic group. For example, a third monomer can include an anionic organosilane such as 3-Trihydroxysilyl propyl methylphosphonate or 3-((trihydroxysilyl)-1-propanesulfonic acid) or cationic organosilanes (e.g., aminopropyltrimethoxysilane). For these embodiments, the inclusion of charged group can increase the wettability of particles to provide improved incorporation in certain media such as high water-content compositions. Also, the inclusion of charged groups can improve the dispersibility (disaggregation) of the particles in water due at least in part to charge-charge repulsion.

In embodiments of the disclosure, the monolith or a particle derived from the monolith can have a binding capacity for a compound or a precursor. In an example embodiment, the compound can include nitric oxide (NO) and the binding capacity for NO can be between about 850 to about 3200 nmol NO per mg dried monolith or per mg dried material (e.g., particles) derived from the monolith. In certain embodiments, the binding capacity for NO can be about

1800 to about 1100 nmol NO per mg monolith.

For embodiments of the disclosure, the compound or precursor may be present as a chemically bonded or physically absorbed species. As an example, NO may be chemically bonded to the sulfur group present on the first monomer to form an S-nitroso linkage. Methods for the preparation of S-nitroso linkages are described in further detail in the remainder of the disclosure.

In certain embodiments, the monolith and particles derived from the monolith can include a porous structure characterized by a pore size and a pore volume. In some embodiments, the porous structure can be adjusted using different combinations of first monomer and second monomer. For certain applications, the use of a porous particle may provide an advantage by having a greater surface area for binding the compound (e.g., NO) and/or modifying the delivery kinetics.

In an embodiment of the disclosure, the monolith can have a framework that includes a pre-gel, a gel (e.g., a hydrogel), a xerogel, or an aerogel. As used herein, a pre-gel is meant to describe a precursor to a gel, where a short-range framework has started to form, but the framework is not self-supporting. A pre-gel can be visually and physically identified using tests disclosed herein. For example, a pre-gel generally displays precipitation (e.g., cloudiness) in solution. A pre-gel also generally displays flow and/or disruption when inverted. A gel may be distinguished from a pre-gel in that the gel does not display flow or disruption when inverted, a characteristic referred to herein as self-supporting. A xerogel refers to a dry gel that can be produced by evaporating a wetting agent from a gel. For a hydrogel, the wetting agent includes water, though other wetting agents such as alcohols (e.g., methanol, ethanol, propanol, etc.) may be present in the gel. Thus, in embodiments of the disclosure, the monolith can have a framework that includes a gel, a pre-gel, a xerogel, and an aerogel.

In embodiments of the disclosure, the particles derived from the monolith may be derived from a monolith having any framework, from multiple monoliths having the same framework, or from multiple monoliths having different frameworks. Additionally, particles can be derived from multiple monoliths and then combined to form a mixture of particles derived from different monoliths.

Generally, particles derived from a monolith display physical characteristics which can differentiate them from nucleated particles. For example, particles derived from a monolith can include a particle or particles having a rough surface. As used herein, a rough surface indicates that the surface includes a plurality of projections extending from the particle surface, each projection having about a 0.1 $\mu$m to about 3 $\mu$m scale, such as about 0.4 $\mu$m to about 2.5 $\mu$m, about 0.8 $\mu$m to about 2.2, and about 1 $\mu$m to about 2 $\mu$m. In some embodiments, the particle having a rough surface can include a particle derived from a monolith.

The rough surface of the particles can be illustrated by the BET surface area of the particles. A particle with greater roughness generally will have a higher BET surface area. The particles of the present disclosure, for instance, can have a BET surface area of greater than about 5 $m^2/g$, such as greater than about 7 $m^2/g$, such as greater than about 9 $m^2/g$, such as greater than about 11 $m^2/g$, such as greater than about 13 $m^2/g$, such as greater than about 15 $m^2/g$, and generally less than about 100 $m^2/g$.

Example aspects of particles disclosed herein, including particles derived from a monolith and particles having a rough surface, include a particle size between about 0.1 to about 100 microns. The average particle size, for instance, can be greater than about 0.5 microns, such as greater than about 1 micron, such as greater than about 1.5 microns, and generally less than about 80 microns, such as less than 60 microns, such as less than 40 microns, such as less than about 30 microns, such as less than about 20 microns, such as less than about 10 microns, such as less than about 8 microns, such as less than about 5 microns. Particle size can be measured using any suitable light scattering or laser method. Additionally, these particles include a first monomer, as well as a second monomer as described in embodiments of the monolith.

In an example embodiment, a plurality of particles can include a group of particles each having a rough surface. In certain embodiments, the group of particles may be polydisperse. Alternatively, in some embodiments, the group of particles may be monodisperse. To achieve a certain size range or particle distribution, in some embodiments, particles derived from a monolith may undergo additional processing, such as milling using a horizontal bead mill. For example, a planetary ball mill or a horizontal bead mill can be used to wet mill the particles. Wet milling involves mixing the particles with an appropriate liquid (typically water, alcohol, or water/alcohol mix; in some cases, an acid, base, or buffer can also be included to adjust the pH to improve milling efficiency) in a milling chamber with milling beads (chamber and beads typically are of matched materials with high density and high hardness, e.g. $ZrO_2$; diameter of beads is in the range of 0.1-1 mm). High speed rotation of the milling chamber generates high impact force to reduce particle size to the submicron range. Smaller beads and longer milling times produce smaller particles. For some applications, the particles may be milled down to a diameter of about 100 nm or less.

An example method for determining polydispersity for a mixture of particles can include Eq. 1 in which dispersity, Đ, is calculated using the standard deviation (SD) and mean particle size (mean) as shown below:

$$\text{Dispersity } Đ = [SD/mean]^{\wedge}2 \qquad \text{Eq. 1}$$

Đ < 0.05; very monodisperse
Đ < 0.08; nearly monodisperse.
Đ = 0.08 - 0.7; mid-range value.
Đ > 0.7; very broad distribution of particle sizes.

Controlling particle size can be used to modify the particle loading capacity by increasing the surface area relative to the particle mass. Particle size may also impact the penetration of particles into the skin. Thus, for some embodiments, methods for forming a particle may also include milling the particles to adjust the average particle size (e.g., reducing the average particle size).

In another example embodiment, the particle or particles having a rough surface can include a density of sulfur groups between about 1500 μmol to about 3000 μmol per mg particle.

Additional embodiments of the disclosure can include methods for producing a monolith. These methods can include hydrolyzing a first monomer in a solution having a pH less than or equal to 7, providing a second monomer to the solution, reacting the first monomer and the second monomer for a reaction time at a reaction temperature, and providing a base to the solution at the end of the reaction time. Where, providing the base to the solution increases the solution pH to a final pH that is greater than the starting pH.

Generally, the first monomer and the second monomer can include the first monomers and second monomers disclosed forming of the monolith structure. For example, the first monomer can include structures derived from Structure I or Structure II, and the second monomer can include structure derived from Structure III. Additionally, the ratio of the first monomer to the second monomer can include the ratios disclosed as forming the monolith structure. For example, the first monomer can be present at a ratio of 1:5 to about 5:1, or a ratio of about 1:4 to about 4:1.

Example aspects of hydrolyzing the first monomer in solution can include a first monomer concentration and a solvent. Several non-limiting examples of solvents that can be included in solutions to produce a monolith include: water; an alcohol (e.g., methanol); a linear or branched alkane (e.g., hexane); dimethyl sulfoxide (DMSO); dimethyl formamide (DMF); tetrahydrofuran (THF); benzene; toluene; and combinations thereof. Additionally, the first monomer concentration can be between about 5 vol% to about 20 vol%, such as about 7 vol% to about 15 vol%, or about 9 vol% to about 12 vol%.

In embodiments of disclosure, the method for producing a monolith can be conducted at a starting pH less than or equal to about 6. In an example embodiment, the method for producing the monolith can be conducted at a starting pH less than or equal to about 3. In another example embodiment, the starting pH can be less than or equal to about 2.

In embodiments of disclosure, the method for producing a monolith can include providing a base to increase the pH to a final pH greater than or equal to about 6. In an example embodiment, the final pH can be greater than or equal to about 7. In another example embodiment, the final pH can be greater than or equal to about 10.

In embodiments of the disclosure, the method for producing a monolith can be conducted at a reaction temperature between about 25 °C and about 80 °C. For instance, an example embodiment of the disclosure can include a method for producing a monolith, where reacting the first monomer with the second monomer occurs at a reaction temperature between about 33 °C and about 60 °C. Additionally, or alternatively, the method for producing a monolith can be conducted for a reaction time between about 0.5 hour and about 8 hours. For instance, an example embodiment of the disclosure can include reacting the first monomer and the second monomer for a reaction time between about 1 hour and about 5 hours.

In an example embodiment, the method for producing a monolith can further include determining the reaction time using an inversion test. For this implementation, the inversion test can include reacting the first monomer and the second monomer for a timespan in a reaction vessel, inverting the reaction vessel upon reaching the timespan, or determining whether the self-supporting structure is formed based at least in part on if the monolith holds in the reaction vessel upon inverting the reaction vessel. In certain implementations, it may be useful to iteratively perform the inversion test to determine whether the timespan can be longer or shorter. For these implementations, determining whether the self-supporting structure is formed can include a decision based on if the monolith holds during inversion. If the monolith holds, the timespan can be decreased and the inversion test repeated. Otherwise (i.e., if the monolith does not hold), the timespan can be increased and the inversion test repeated. In some implementations, the number of iterations can be set. In some implementations, the number of iterations can be based on a convergence (e.g., if in the prior iteration the timespan was increased and in the current iteration the timespan was decreased, then the test has converged). Additionally, the amount by which the timespan is increased or decreased may be static or can change and thus is not intended to be limited to only a single value.

Another embodiment of the disclosure can include a method for forming a plurality of particles, each having a

rough surface, the method including mechanically disrupting a 3-dimensional structure, where the 3-dimensional structure includes a first monomer and a second monomer linked to form a framework.

For certain implementations, mechanically disrupting the 3-dimensional structure can include vortexing a solution containing the 3-dimensional structure, grinding the 3-dimensional structure in either a gel form in solution or in a xerogel form not in solution, or combinations of these. As an example, a monolith gel formed in solution, as described in example embodiments herein, can be vortexed to form a plurality of polydisperse particles. As used herein, vortexing can be accomplished by a device or implement that can generate currents in the solution to agitate the solution in contact with the monolith. As used herein, grinding can be accomplished by a device or implement that directly contacts the monolith to disturb or modify the 3-dimensional structure. Thus, disrupting the 3-dimensional structure need not require directly contacting the monolith and can include agitating a fluid surrounding the monolith and/or directly applying force to the monolith itself.

Generally, mechanically disrupting the 3-dimensional structure produces a polydisperse mixture of particles having a rough surface. In certain implementations, it may be advantageous to separate the polydisperse particles to form a group of monodisperse particles each having a rough surface. Example methods separating a polydisperse mixture can include sedimentation, which can be used in combination with fluid flow so that lighter particles are carried further along the direction of fluid flow compared to heavier particle. Additionally, or alternatively, a sieve having size selective holes may be used to separate the polydisperse mixture.

Another example embodiment of the disclosure includes a method for loading the particles having a rough surface. Example embodiments for loading the particles having a rough surface may include proving a plurality of particles derived from a monolith with a compound or a precursor, the precursor undergoing conversion to the compound on exposure to a condition.

In implementations for loading the particles having a rough surface, the particles may be present in a solution containing the compound or the precursor. In certain implementations, the concentration of the compound or the precursor can be about 5 wt% to about 30 wt%. In an example implementation, the concentration of the compound or the precursor can be about 10 wt% to about 15 wt%.

Additionally, an aspect loading the particles having a rough surface can include a loading temperature. In an example implementation, the loading temperature can be between about 0 °C to about 40 °C. In another example implementation, the loading temperature can be between about 15 °C to about 25 °C.

In another embodiment of the disclosure, the precursor can be a nitrite or a nitrite-containing compound. Several non-limiting examples of the nitrite or nitrite-containing compound may include nitroglycerine or a nitrite salt, such as sodium nitrite or potassium nitrite.

In an embodiment of the disclosure, the condition for converting the precursor to the compound can include an acid. In an implementation, the acid can include an organic acid (e.g., glycolic acid, acetic acid, tartaric acid, or lactic acid). Additionally, or alternatively, the acid can include an inorganic acid (e.g., hydrochloric acid, sulfuric acid, phosphoric acid, etc.).

Another example embodiment of the disclosure includes a pharmaceutical and/or cosmetic composition, including a medium containing a plurality of particles loaded with a compound or precursor. In an example embodiment, the plurality of particles loaded with a compound or precursor can be formed from particles derived from a monolith as described in embodiments of the disclosure. In certain implementations, the medium can include a petroleum product. Several non-limiting examples of the petroleum product include: methylparaben, mineral oil, isopropyl myristate, white petrolatum, emulsifying wax, and propylparaben, or combinations thereof. Alternatively, or additionally, in some implementations, the medium can include a natural product. Several non-limiting examples of the natural product include bee's wax, soy wax, a plant-derived oil, cellulose, guar gum, and combinations thereof.

In some implementations, the medium can further include water and a surfactant. In certain implementations the surfactant can be anionic, cationic, zwitterionic, nonionic or a combination of these. Several non-limiting examples of nonionic surfactants that can be used in embodiments of the disclosure include alcohols (e.g., 1-octanol), fatty acids (e.g., palmitic acid), and fatty acid esters (e.g., glycerol monostearate).

Another example embodiment of the disclosure includes a method for delivering a compound to a local area. In an example implementation, the method for delivering a compound to a local area can be used to treat a patient having a disease or a having been diagnosed with a disorder. For instance, an embodiment of the disclosure can include a method for treating a patient having been diagnosed with a disorder, the method including administering a composition containing a plurality of particles having a rough surface to a local area on the patient. In an example implementation, the plurality of particles can be loaded with a compound or a precursor, such that administering the composition to the local area triggers a release of the compound. In certain implementations, the release may be substantially instantaneous, such that at least 50% of the compound is delivered in less than 60 seconds. In some implementations, the release may be extended, such that at least 50% of the compound is delivered in less than 300 seconds.

To achieve substantially instantaneous release, an accelerator may be used in combination with administering the composition containing a plurality of particles loaded with the compound.

Generally, embodiments of the disclosure can be combined or used in conjunction with other embodiments disclosed herein. For example, the composition containing plurality particles having a rough surface can include particles derived from a monolith, the monolith including a first monomer and a second monomer. Additionally, the particles can be loaded using any of the methods described for including a compound or a precursor that can be physically or chemically associated with a portion of the particle.

In an example implementation, the local area can include a portion of the patient's body, such as the skin (e.g., the face, torso, limbs, hands, nails, and feet) and/or a mucous membrane (e.g., the mouth, the sinus, the rectum, and the vagina). While some particles may dislodge or otherwise removed from the local area, aspects of certain embodiments of the disclosure can result in the improved retention of most particles. For example, compositions including particles having a rough surface, can imbed in the surface of soft tissue which can provide benefits for treating local rather than systemic disorders and diseases, or for providing antibacterial agents at a site of infection or a site of contamination.

In embodiments of the disclosure that include methods for delivering a compound, the particles having a rough surface can further include a release rate for the compound, thereby delivering an amount of the compound over time. In an example implementation, the amount of the compound can be released over a timespan. For instance, 85% of the compound can be released after 36 hours. Thus, for this example, the amount of compound is 85% based on the amount contained in the particles before administration and the timespan is 36 hours. In embodiments of the disclosure, the amount of the compound can be from about 5% to about 100% (complete delivery) and the timespan can be from about 0.5 hour to about 36 hours.

In some instances, the release rate can be modified or adjusted using a release condition. For example, Figs. 4A and 4B display graphs showing the instantaneous release of NO and the cumulative release of NO, respectively. These graphs were determined under accelerated conditions (e.g., exposure to light, acid, and/or heat) and in aqueous solution. In contrast, FIGs. 13A, 13B, 15A, and 15B were obtained using physiologically relevant conditions.

In some embodiments, delivering the compound can include delivering NO using a plurality of particles having a rough surface. For instance, an example embodiment can include administering a composition containing plurality of particles loaded with NO to a patient, the particles having a release rate such that 85% of the NO is released after about 36 hours. Another example embodiment can include administering a composition containing a plurality of particles loaded with NO to a patient, the particles having a release rate such that at least 50% of the NO is released after about 10 hours. An additional example embodiment can include administering a composition containing a plurality of particles loaded with NO to a patient, the particles having a release rate such that at least 25% of the NO is released after about 3 hours.

Certain benefits may be recognized by administering the particles to a site, including the skin or a mucous membrane, without requiring the use of a patch. Generally, the particles described herein can be delivered to a subject by a variety of topical or systemic routes of delivery, including but not limited to, percutaneous, inhalation, oral, local injection and intravenous introduction. The particles can be incorporated, for example, in a cream, ointment, transdermal patch, implantable biomedical device or scrub.

An additional aspect of the disclosure can include a method for treating an infection in a subject, the method including administering to the subject a particle loaded with NO or a composition including the same in an amount and manner effective to treat the infection. Depending on the site of the infection, the particles can be administered topically or systemically.

The term "infection" is used to include infections that can produce an infectious disease. The infectious diseases may include communicable diseases and contagious diseases. As used herein, treating an infection can mean eliminating the infection, reducing the size of the infection, preventing the infection from spreading in the subject, or reducing further spread of the infection in the subject.

The infection can be, for example, a bacterial, viral, fungal or parasitic infection. The bacterial infection can be a *Staphylococcal* infection. The bacterial infection can be caused, for example, by a bacterium such as *S. aureus*, Multidrug-resistant or Methicillin-resistant *S. aureus* (MRSA), *P. aeruginosa, B. circulans, B. cerius, E. coli, P. vulgaris, P. acnes, S. pyognenus, S. enterica, V. angulillarum*, *K. pneumoniae*, *P. piscicida*, *P. aeruginosa*, *A. tumefaciens*, *C. micgiganence*, *A. mali, E. chourysanthemi, X. campestris, C. diplodiella, P. piricoloa*, *M. tuberculosis,* and *M. ulcerans.* The fungal infection can be caused, for example, by a fungus such as *T. equinum*, *C. Albicans*, *F. oxysporum*, *R. solani, B. cinereal*, *T. rubrum,* and *A. flavus.* The viral infection can be caused, for example, by a virus such as *M. contagiosum*, *Rota, Papilloma*, *Parvo*, and *Varicella.* The parasite infection can be caused, for example, by a parasite of the genus *Plasmodium*, *Leishmania, Schistosoma*, *Austrobilharzia*, *Heterobilharzia*, *Ornithobilharzia,* or *Cryptosporidium*, for example *P. falciparum.*

An additional aspect of the disclosure can include a method for promoting angiogenesis, vasodilation, smooth muscle relaxation, wound healing, erectile function, or hair growth in a subject, the method including administering to the subject a particle loaded with NO or a composition including the same in an amount and manner effective to promote angiogenesis, vasodilation, smooth muscle relaxation, wound healing, erectile function or hair growth

A further aspect of the disclosure can include a method for treating a disease or disorder in a subject the method

including administering to the subject a particle loaded with NO or a composition including the same in an amount effective to treat hypertension, peripheral vascular disease, platelet aggregation, erectile dysfunction, ischemia, inflammation, a wound, an abscess, scleroderma, and sickle cell anemia.

EXAMPLE 1

Example 1 discusses various methods and procedures and provides exemplary embodiments that may be understood in conjunction with the Drawings and Description provided herein. It should be understood that the disclosure is not limited solely to the examples provided, and that the conditions and materials disclosed are exemplary.

METHODS

*Two-pot Silica Gel Monolith Synthesis*

A silica sol-gel monolith containing mercaptopropyl functional groups was synthesized by performing independent hydrolysis of silica monomers tetraethoxysilane (TEOS) and 3-mercaptoproyltrimethoxysilane (MPTS), followed by monomer condensation to form a spanning network. Following condensation, the monolith was dispersed into discrete silica particles by application to a vortex mixer on high setting (this mixing process is hereafter referred to as "vortex" or "vortexing"). In this example, the mole % of MPTS was 37.4% (balance TEOS), but sol-gel monoliths of differing mechanical properties can be formed with MPTS mole % in the range of 5%-80% (balance TEOS). Varying the monolith strength in this manner can be employed as a means of influencing the particle conversion process yielding varying mean particle size and size distribution.

The MPTS and TEOS hydrolysis reactions proceed for different lengths of time, so the start of each reaction was coordinated such that both hydrolysis reactions ended at the same time. MPTS hydrolysis was initiated by combining the following: 3.2 milliliters (ml) Methanol; 0.655 ml deionized (DI) HzO; 0.153 ml 0.1N hydrochloric acid (HCl); and 0.4 ml MPTS, and vortexing to mix well. The MPTS hydrolysis reaction was allowed to proceed at room temperature (RT) undisturbed for 90 minutes. TEOS hydrolysis was initiated 30 minutes after starting MPTS hydrolysis by combining the following in a separate vessel: 1.24 ml anhydrous Ethanol; 0.655 ml DI $H_2O$; 0.073 ml 0.1N HCl; 0.8 mL TEOS; and vortexing to mix well. The TEOS hydrolysis reaction was allowed to proceed undisturbed for 60 minutes at RT. The end of each hydrolysis reaction occurred at the same time. At the end of hydrolysis, the MPTS and TEOS hydrolysates were combined and vortexed to mix. Then, condensation (sol-gel monolithic spanning network formation) was initiated by adding 5.8 ml of sodium phosphate buffer (0.1 M, pH 7.4) and vortexing to mix well. The monolithic spanning network formed after approximately 5 minutes, as evidenced by a change in color from clear to cloudy to opaque white (FIGs. 1A-D). The vessel containing the sol-gel monolith was transferred to a 40°C water bath 15 minutes after starting condensation and remained there undisturbed for 4 hours until condensation was complete. The silica sol-gel monolith was subsequently dispersed into particles by vortexing to disrupt the gel, transforming the material into a viscous suspension of particles (FIGs. 1G-H). The dispersed particles were washed with deionized $H_2O$ 3 times by dispersing in water and vacuum filtering in a Buchner funnel with Whatman Grade 1 Filter paper. Washing the particles, however, is optional. After the third filtration, the product was vacuum dried at room temperature for at least 2 hours or until completely dry, as indicated by a lack of change in weight over a 30-minute time period. After drying is complete, the product was recovered from the filter paper and weighed to assess yield, followed by storage at RT until characterization or use.

The synthesized silica particles were characterized in several ways. Scanning electron microscopy (SEM) was utilized to visualize particle morphology and determine approximate size (FIGs. 2A-2C). SEM images reveal aggregated particles with a relatively narrow size distribution and irregular rounded, but not spherical morphology. It was noted that these particles disperse further upon resuspension. The particle size distribution of hydrated and dispersed particles was quantitatively determined using a laser diffraction particle size analyzer (Beckman Coulter LS 13 320), showing a mean particle size of around 3 $\mu$m (FIG. 3). Nitric oxide (NO) capacity was determined by forming R-SNO groups on particles by mixing dispersed particles with a molar equivalent of sodium nitrite and 1 molar hydrochloric acid, followed by measurement on an EcoPhysics CLD60 chemiluminescence NO analyzer. The NO load was determined as the moles of NO released per mg of particles. The NO release profile and cumulative release curves are shown in FIGs. 4A-4B. The total NO loading efficiency was determined to be 50-63% of theoretical thiol load or up to ~2.8 $\mu$mol NO per mg particle. A list of relevant values for particle size, yield, and NO load for 4 batches of SNO particle synthesis can be found in Table 1.

Table 1. Example particle properties.

| | Batch 1 | Batch 2 | Batch 3 | Batch 4 |
|---|---|---|---|---|
| Mean ($\mu$m) | 3.04 | 3.13 | 2.36 | 3.28 |

(continued)

| | Batch 1 | Batch 2 | Batch 3 | Batch 4 |
|---|---|---|---|---|
| Median (μm) | 2.66 | 2.49 | 1.94 | 2.82 |
| S.D. (μm) | 2.12 | 2.52 | 1.65 | 2.32 |
| Span (μm) | 2.07 | 2.38 | 2.16 | 2.12 |
| Yield (%) | 91.6 | 98.2 | 85.2 | 89.2 |
| NO load (nmol NO/mg) | 2781 | 2867 | 2375 | 2272 |

*One-pot Silica Gel Synthesis*

Reagent amounts and conditions were identical to those described in the two-pot silica gel synthesis, except the reagents for TEOS hydrolysis were added directly to the vessel in which MPTS hydrolysis was underway, and the phosphate buffer was added directly to this vessel to start condensation. With 37.4% MPTS (balance TEOS), one-pot synthesis resulted in silica particles with similar yield, particle size distribution, and S-nitrosation efficiency to those particles synthesized by a two-pot synthesis approach. Mean values for yield, particle size, and NO capacity for n=3 batches each of two-pot and one-pot synthesis are compared in Table 2. This demonstrates that a one-pot approach may be taken in synthesizing silica sol-gel monoliths to generate mercaptopropyl-functionalized particles with similar properties to a standard two-pot approach.

Table 2. Example particle properties using a one pot and a two-pot synthesis.

| | Two Pot | One Pot |
|---|---|---|
| Mean (μm) | 2.92 | 3.43 |
| Median (μm) | 2.42 | 2.97 |
| S.D. (μm) | 2.17 | 2.44 |
| Span (μm) | 2.22 | 2.13 |
| Yield (%) | 90.9 | 94.3 |
| NO load (nmol NO/mg) | 2504 | 2391 |

*Variation of condensation/gel aging time and temperature*

The sol-gel condensation/aging time was systematically varied to determine its effect on sol-gel monolith stiffness and the resulting yield, particle size, and NO loading capacity at two condensation temperatures - room temperature and 40°C. Mercaptopropyl-functionalized silica sol-gel monoliths were synthesized using the two-pot approach described in Example 1, except the condensation/gel time was systematically varied from 30 minutes to 96 hours, and carried out at room temperature (RT) or 40°C. Sol-gel monoliths were formed and condensation proceeded for the following times prior to dispersion into particles: 30 minutes, 1 hour, 90 minutes, 2 hours, 4 hours, 24 hours, 96 hours. Gel stiffness was assessed at each timepoint by inverting the vessel containing the monolith and observing whether the gel remains suspended in the bottom of the tube. At each timepoint, the gel was dispersed, washed/filtered and dried prior to analysis. Condensation at RT resulted in a notably stiffer gel than condensation at 40°C, which was most apparent at early condensation times, but was noticeable for all timepoints (FIGs. 5A-5J). While condensation at 40°C yielded a gel even after 30 minutes of condensation, the gel was not as stiff and would not remain suspended in the top of the tube until condensation times of 4 hours. Even for 4 hours or longer condensation, minimal shaking of the tube was sufficient to cause the gel to fall, indicating a weaker gel.

Yield of particles was high from early condensation times increasing marginally with condensation time up to -95% (FIG. 6A). Particle size analysis indicated that for both condensation temperatures, particle size decreased with condensation time until size plateaued around 3 μm diameter (FIG. 6B). Notably, this plateau was reached at much shorter condensation times at 40°C than at RT, indicating that while particle size and gel stiffness change with condensation time, stiffer gels do not necessarily lead to smaller particles. Additionally, analysis of NO loading and release demonstrated that NO loading increases with condensation time with a plateau near 2.8 μmol NO/mg particles (FIG. 6C). As with particle size, this plateau is reached at shorter condensation times at 40°C than at RT, indicating that condensation temperature can influence NO loading. The NO loading results indicate that consistent with literature, gel

aging is a dynamic process and that the fraction of MPTS that becomes incorporated into the gel prior to disruption into particles continues to change over time. Data for size statistics, yield, and nitric oxide load are tabulated for all samples in Table 3.

Table 3. Example particle properties at different reaction temperatures (temp) and condensation times (time).

| Temp | Time (hour) | Mean (μm) | Median (μm) | S.D. (μm) | Span (μm) | Yield (%) | NO load (nmol NO/mg) |
|---|---|---|---|---|---|---|---|
| RT | 0.5 | 16.61 | 5.68 | 20.42 | 8.80 | 69.4% | 896 |
| RT | 1.0 | 6.49 | 3.45 | 8.44 | 4.68 | 61.8% | 2018 |
| RT | 1.5 | 5.43 | 2.84 | 7.93 | 4.32 | 58.4% | 2019 |
| RT | 2.0 | 4.22 | 2.65 | 4.75 | 3.57 | 82.6% | 2300 |
| RT | 4.0 | 3.96 | 2.46 | 5.50 | 2.92 | 79.6% | 2156 |
| RT | 24 | 2.95 | 2.40 | 2.22 | 2.34 | 94.4% | 2400 |
| RT | 96 | 2.66 | 2.31 | 1.83 | 2.04 | 91.0% | 2770 |
| 40 °C | 0.5 | 4.30 | 2.76 | 6.08 | 2.85 | 62.0% | 1701 |
| 40 °C | 1.0 | 3.07 | 2.65 | 2.20 | 2.15 | 75.4% | 2396 |
| 40 °C | 1.5 | 2.83 | 2.46 | 1.97 | 2.09 | 78.8% | 2575 |
| 40 °C | 2.0 | 3.04 | 2.47 | 2.31 | 2.38 | 79.8% | 2338 |
| 40 °C | 4.0 | 3.13 | 2.49 | 2.52 | 2.38 | 98.2% | 2867 |
| 40 °C | 24 | 2.98 | 2.59 | 2.08 | 2.09 | 89.8% | 2521 |
| 40 °C | 96 | 2.85 | 2.48 | 1.97 | 2.06 | 85.8% | 2714 |

*Effect ofMPTS/TEOS ratio on sol-gel monolith properties*

The range of MPTS/TEOS ratios under which sol-gel monoliths form and the properties of the resultant monoliths were investigated. Sol-gel monoliths were synthesized by two pot synthesis according to Example 1, but the ratios of MPTS and TEOS were varied. The mole % of MPTS was varied from 10% to 80% (balance TEOS). Sol-gel monoliths formed for MPTS mole percentages of 0-60% were evaluated. MPTS fraction of 0-30% yielded stiffer, clearer sol-gel monoliths that did not disperse upon vortex mixing. The color became increasingly clear as MPTS fraction decreased. MPTS fraction of 37-60% yielded opaque, white sol-gel monoliths of varying stiffness, where stiffness decreases with increasing MPTS. Note that only MPTS fractions of 35-40% formed a typical, relatively stiff sol-gel, with increasing MPTS fractions yielding dense but weaker gels.

Monoliths were dispersed into particles by different methods depending on the respective monolith properties. Sol-gel monoliths in the 37-60% MPTS range that formed an opaque, white monolith were dispersed by vortexing and triple-washed/filtered prior to drying. Monoliths that formed hard, clearer gels were unable to be dispersed by vortexing alone. MPTS fraction of 0-10% was manually broken up with a spatula prior to rinsing and drying. MPTS fractions of 20-30% required addition of deionized (DI) $H_2O$ and shaking prior to vortexing in order to disperse. Sol-gels with MPTS fraction that did not yield a monolith were also vortexed to mix, then triple-washed and dried.

FIG. 11 contains resultant particle characterization outcomes. The yield for each MPTS/TEOS ratio was calculated and found to be similarly high, regardless of monolith properties. However, particle size varied significantly. For stiffer monoliths, particle size was larger (~250 μm) and particles derived from the monoliths demonstrated more granular and glassy characteristics. In the MPTS/TEOS range where a monolith did not form (MPTS > 60%), the resultant product was dense, sticky, and unable to be dispersed into particles and so were not further analyzed.

NO loading and nitrosation efficiency were also evaluated. NO load increased with increasing MPTS fraction in the range 5-40% MPTS, but then decreased above 40. This demonstrates that MPTS/TEOS ratios may be selected to achieve varying monolith and resulting particle properties, which also translates into differences in NO loading.

*NO release using simulated skin conditions*

NO release from compositions including particles loaded with NO was evaluated to simulate the spreading that would occur when applied topically. For this simulation, a composition including dry particles loaded with NO was

incorporated into a petroleum vehicle. The composition included dry S-nitrosated particles mixed with an anhydrous petrolatum delivery vehicle, the particles present at 25% by weight of the composition. The composition was then immediately applied to a piece of wax paper having a surface area of 6 $cm^2$ and spread thinly to simulate topical application. The wax paper was then transferred to a sampling chamber held at 34 °C and NO release was monitored. The sampling chamber was isolated from outside lighting and placed under a high precision LED microscope white light lamp with adjustable setting (AmScopeLED-6WD); lux was measured using a digital lux meter (Dr. Meter, Model: LX1010B). Levels of nitric oxide were measured every minute for a period of 60 hours.

*NO release in aqueous conditions*

NO release in aqueous conditions was evaluated to simulate alternative delivery routes, such as intravenous administration. For this, a 50 mg aliquot of S-nitrosated particles was added to 5 mL of phosphate buffer in a sampling chamber at 37 °C which was connected to a nitric oxide analyzer. Levels of nitric oxide were measured every 30 seconds for a period of 12 hours.

RESULTS

Results provided in the drawings and described herein are meant to be exemplary and are not intended to limit the methods and compositions to modifications or alternatives as would be understood by a person of ordinary skill in the field of endeavor.

Referring now to FIGs. 1A-1H illustrating the progression of sol-gel monolith condensation. The images shown images are at (A) 1 minute, (B) 3 minutes, (C) 5 minutes, (D) 10 minutes after start of condensation. At end of condensation (4 hours at 40°C), sol-gel monolith is opaque white and holds in the tube when inverted (E-F). Disruption of the sol-gel by vortexing dispersed the monolith into particles (G-H), which takes on liquid form.

Referring now to FIGs. 2A-2C illustrating scanning electron microscope (SEM) images of particles derived from a monolith. The images are shown at various magnifications, including (A) 1500x, (B) 4000x, and (C) 60000x magnification. The particles shown in the images are irregular and display rough surfaces that include approximately micron scale projections.

Referring now to FIG. 3 illustrating data characterizing example particles derived from a monolith. The graph displays a size distribution from 0.017 μm to 2000 μm. Further, the distribution displays a mean size of 2.956 μm a median size of 2.561, a standard deviation (SD) of 2.030 μm, a lower $10^{th}$ percentile of 0.661 μm, a $50^{th}$ percentile of 2.561 μm, and an upper $90^{th}$ percentile of 5.901 μm.

Referring now to FIGs. 4A and 4B, the graphs shown display example release profiles measuring (A) instantaneous NO release (ppm) and (B) cumulative NO release (nmol/mg particle) for exemplary nitrosylated silica particles derived from a monolith. These release profiles were obtained under accelerated conditions in solution using heat and light. For these graphs, after the samples were prepared, the solutions were placed in a chamber for monitoring NO concentration. The chamber was illuminated with bright which light and the chamber temperature was maintained at 70 °C.

Referring now to FIGs. 5A-5J, the images shown display photographs of sol-gel monolith progression over time at (A-E) 0 °C or (F-J) 40 °C. The gel stiffens quicker at room temperature than at 40° C based on the inversion test.

Referring now to FIGs. 6A-6C, the graphs shown illustrate silica particle (A) yield as a function of condensation time and temperature, (B) size as a function of condensation time and temperature, and (C) NO capacity of S-nitrosylated particles prepared using different condensation times and temperatures.

Referring now to FIGs. 7A-7C, the images shown illustrate whole skin grayscale images demonstrating application of a petroleum-based medium including fluorescently labeled silica particles onto cadaveric human skin for (A) medium alone and (B-C) medium with sulfur functionalized silica particles. FIGs. 7D-7F respectively illustrate FIGs. 7A-7C in color.

Referring now to FIGs. 8A-8C, the images shown illustrate a tissue cross section for samples shown respectively in FIGs. 7A-C demonstrating skin penetration in (A) bright field, (B) fluorescence, and (C) an overlay of FIGs. 8B and 8C. FIGs. 8D-8F respectively illustrate FIGs. 8A-8C in color.

Referring now to FIG. 9, the image shows a graph tracking fluorescence intensity for fluorescently labeled silica particles derived from a monolith which have been applied to human skin. The graph measured detectable fluorescence after the skin region has undergone tape stripping for a number of rounds. While the application and removal of a tape strip is able to reduce the fluorescence, the data demonstrates that fluorescently labeled particles can still be detected after 10 rounds of tape stripping, indicating the imbedding of some particles in the upper skin layer.

Referring now to FIG. 10, the image shows a BET analysis for silica nanoparticles derived from a monolith. The analysis was conducted using nitrogen gas using an Autosorb● iQ Station 1 device. Further information related to FIG. 10 is included in FIG. 11, which includes a summary of data reduction parameters, multi-point BET data, and a summary.

Referring now to FIG. 12, the image shows a table providing information characterizing particles formed from a monolith made using the ratio of MPTS/TEOS shown in the table.

Referring now to FIGs. 13A and 13B, these images illustrate graphs displaying the concentration of NO ppm and NO release rate, respectively, versus time after one-time application of 150 mg ointment containing 25 % by weight of particles loaded with NO. As shown the release displays an approximately exponential decrease, with detectable concentrations of NO present even after 48 hours. FIGs. 14A and 14B, illustrate graphs illustrating that the release profile can be reproduced by continuous application of freshly prepared ointment every 24 hours for up to 144 hours, though likely this trend could be reproduced over as many applications as needed. While these experiments were conducted with freshly prepared ointment, after nitrosated particles are incorporated into ointment, the ointment can be stored in a freezer held at 0 °C for at least 3 months without significant loss of NO.

**[0091]** Referring now to FIGs. 15A and 15B, these images illustrate graphs displaying the NO release rate and cumulative release as measured using the conditions described in NO release using simulated skin conditions section of the methods. The graphs illustrate that at physiologically relevant conditions, controlled NO release can be achieved over an extended period of time, with the majority (50% or more) of NO being released within the first 12 hours.

Referring now to FIG. 16, the image illustrates a graph displaying the cumulative NO release as measuring using the conditions described in the NO release in aqueous conditions section of the methods. The graph illustrates that in solution, NO releases faster compared to topical administration with the majority (50% or more) of NO being released within the first 3 hours.

The application includes the following clauses:

1. A pharmaceutical composition comprising a medium containing a plurality of particles loaded with nitric oxide or a precursor for forming nitric oxide, wherein the plurality of particles comprise:
at least one particle having a rough surface and a functional group, wherein the rough surface comprises a plurality of projections each having a size between about 0.01 μm and 2 μm.

2. The pharmaceutical composition of clause 1, wherein the medium comprises a petroleum product.

3. The pharmaceutical composition of clause 2, wherein the petroleum product includes a product from the group consisting of: methylparaben, mineral oil, isopropyl myristate, white petrolatum, emulsifying wax, and propylparaben.

4. The pharmaceutical composition of any one preceding clause, wherein the medium further comprises water and an emulsifying agent.

5. The pharmaceutical composition of clause 4, wherein the emulsifying agent comprises an alcohol, an anionic surfactant, a cationic surfactant, a zwitterionic surfactant, or combinations thereof.

6. The pharmaceutical composition of any one preceding clause, wherein said at least one particle having the rough surface was derived by disrupting a monolith.

7. The pharmaceutical composition of any one preceding clause, wherein said at least one particle having the rough surface and the function group comprises a first monomer and a second monomer linked to form a matrix having a surface area.

8. The pharmaceutical composition of clause 7, wherein the first monomer includes the chemical structure:

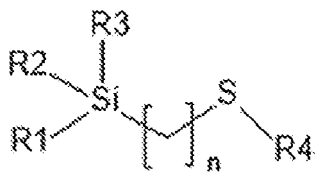

wherein, each of R1, R2, and R3 is independently a hydrogen, a linear or branched alkyl group having 1-5 carbon atoms, or an alkoxy group having 1-5 linear or branched carbon atoms; and wherein R4 is selected from the group consisting of: a hydrogen atom, a linear or branched alkyl group having 1-4 carbon atoms, and n = 1-4.

9. The pharmaceutical composition of any one of clauses 7-8, wherein the second monomer includes the chemical structure:

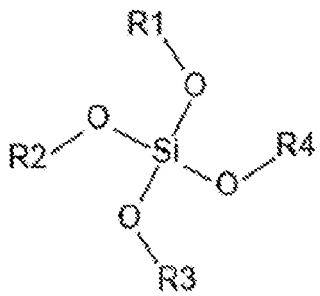

wherein, each of R1-R4 is selected independently from a hydrogen, or a branched or linear alkyl group having 1-5 carbon atoms.

10. The pharmaceutical composition of claim any one preceding clause, wherein the functional group comprises a thiol.

11. The pharmaceutical composition of any one preceding clause, wherein the pharmaceutical composition includes no less than 5.0 wt% and no greater than 33.0 wt% of the plurality of particles loaded with nitric oxide or a precursor for forming nitric oxide based on the total weight of the pharmaceutical composition.

12. The pharmaceutical composition of any one preceding clause, wherein the plurality of particles have a nitric oxide binding capacity no less than about 100 nmol NO and no greater than about 1800 nmol per milligram of the plurality of particles.

13. A method for treating a patient having been diagnosed with a disorder comprising:

administering a composition containing a plurality of particles loaded with nitric oxide or a precursor for forming nitric oxide to an administration site on a patient in need thereof, wherein
at least some of the particles have a rough surface that causes the particles to embed at the administration site.

14. The method of clause 13, wherein the administration site is skin of the patient.

15. The method of any one of clauses 13-14, wherein the compound is released over a timespan.

16. The method of clause 15, wherein at least 85% of the compound is released after 36 hours.

17. The method of clause 15, wherein at least 50% of the compound is released after 10 hours.

18. The method of any one of clauses 13-17, wherein the plurality of particles are loaded with the precursor; further comprising:
delivering a second compound that reacts with the first compound to generate nitric oxide.

19. The method of any one of clauses 13-18, wherein the method does not comprise applying a covering to the administration site.

20. A polydisperse plurality of particles having an average particle size between about 1 μm and about 8 μm, the plurality of particles comprising:

particles having a rough surface and a functional group, wherein, the particles have a BET surface area of greater than about 9 $m^2/g$;
wherein the plurality of particles is derived by disrupting a monolith comprising a first monomer and a second monomer linked to form a framework.

21. A polydisperse plurality of particles as defined in clause 20, wherein the rough surface comprises a plurality of projections each having a size between about 0.1 μm and about 2 μm.

## Claims

1. A method of producing particles, comprising:

   reacting a first monomer with a second monomer,
   wherein:

   the first monomer comprises 3-mercaptoproplytrimethoxysilane,
   the second monomer is selected from among tetraethyl orthosilicate and tetramethyl orthosilicate, and
   a ratio of the molar amount of the first monomer to the molar amount of the second monomer is within a range of from 1:3 to 4:6.

2. The method recited in claim 1, wherein the ratio of the molar amount of the first monomer to the molar amount of the second monomer is within a range of from 35:65 to 40:60.

3. The method recited in clam 2, wherein the second monomer is tetraethyl orthosilicate.

4. The method recited in any one of claims 1 to 3, wherein the method further comprises hydrolyzing at least one of the first monomer and the second monomer.

5. The method recited in any one of claims 1 to 4, wherein the method further comprises adding phosphate.

6. The method recited in any one of claims 1 to 5, wherein the method comprises:

   forming a monolith; and
   breaking at least some of the monolith to form said particles.

7. The method recited in any one of claims 1 to 6, wherein the method further comprises adding a base after said reacting the first monomer and the second monomer,

8. The method recited in any one of claims 1 to 7, wherein said reacting the first monomer with the second monomer is carried out at a temperature between room temperature and about 80° C.

9. The method recited in any one of claims 1 to 8, wherein said reacting the first monomer with the second monomer is carried out for a reaction time between about 0.5 hour and about 96 hours.

10. A composition comprising:
    a plurality of particles produced by a method recited in any one of claims 1 to 9.

11. The composition recited in claim 10, wherein a mean particle size of the plurality of particles is in a range of from 2.36 micrometers to 6 micrometers, preferably, wherein a mean particle size of the plurality of particles is in a range of from 3.0 micrometers to 4.5 micrometers.

12. The composition recited in any one of claims 10 to 11, wherein the composition further comprises at least one compound selected from nitric oxide and compounds that release nitric oxide.

13. The composition recited in any one of claims 10 to 12, wherein the method comprises:

    forming a monolith; and
    breaking at least some of the monolith to form said particles.

14. The composition recited in claim 12 for use as a medicament.

15. The composition recited in claim 12 for use in treatment for providing localized delivery of nitric oxide.

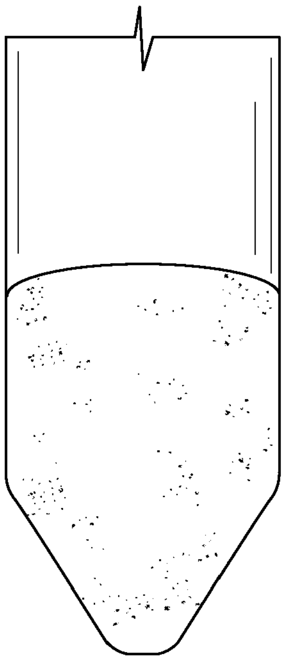

FIG. 1A

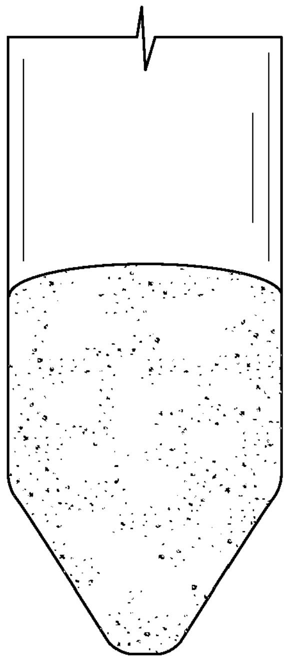

FIG. 1B

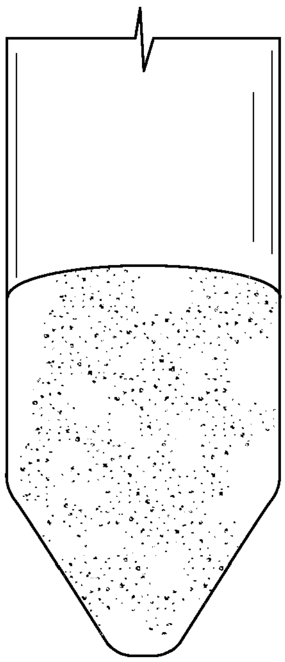

FIG. 1C

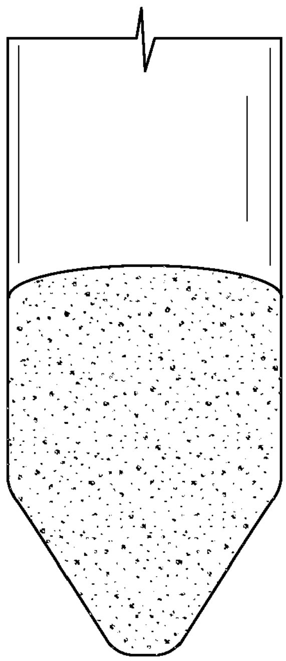

FIG. 1D

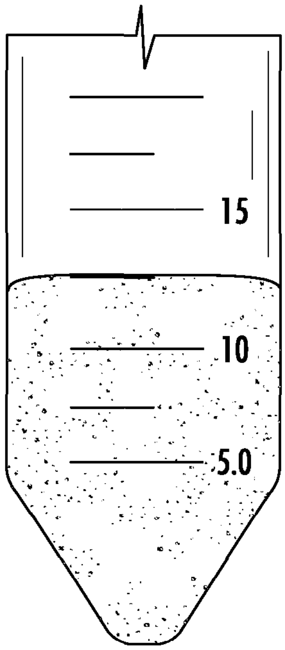


FIG. 1E

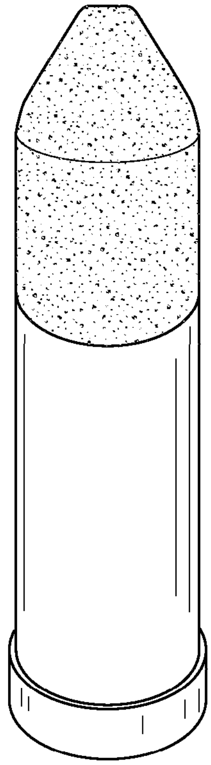

FIG. 1F

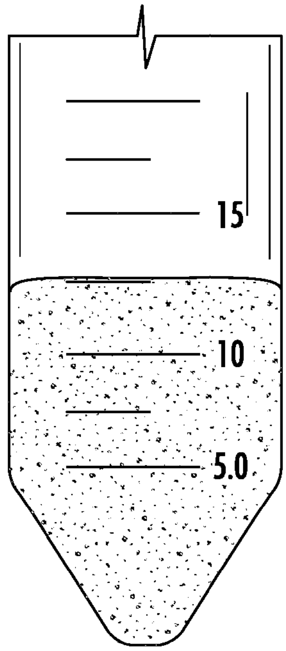


FIG. 1G

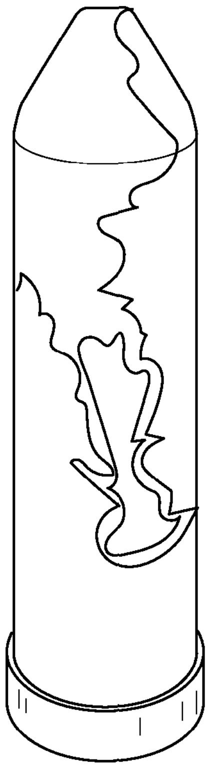

FIG. 1H

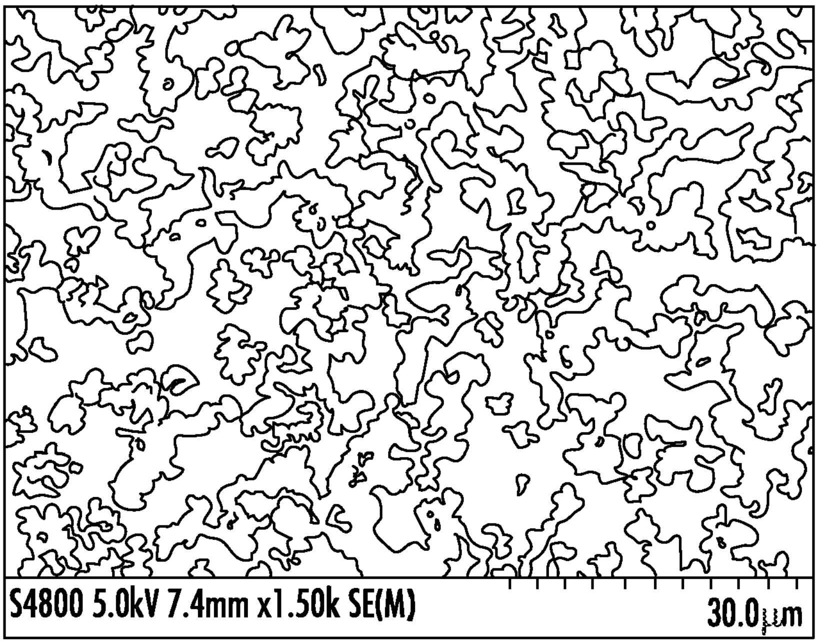
S4800 5.0kV 7.4mm x1.50k SE(M)
30.0μm

FIG. 2A

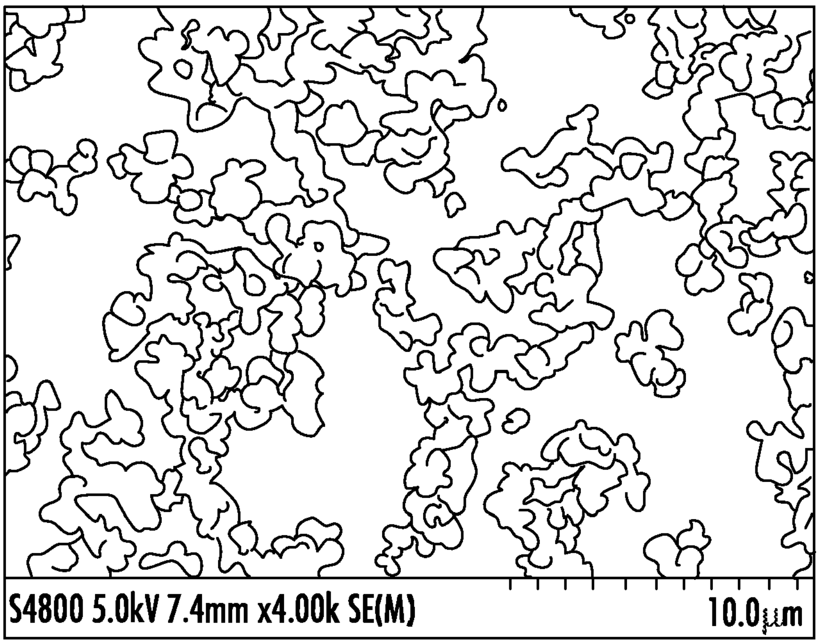
S4800 5.0kV 7.4mm x4.00k SE(M)
10.0μm

FIG. 2B

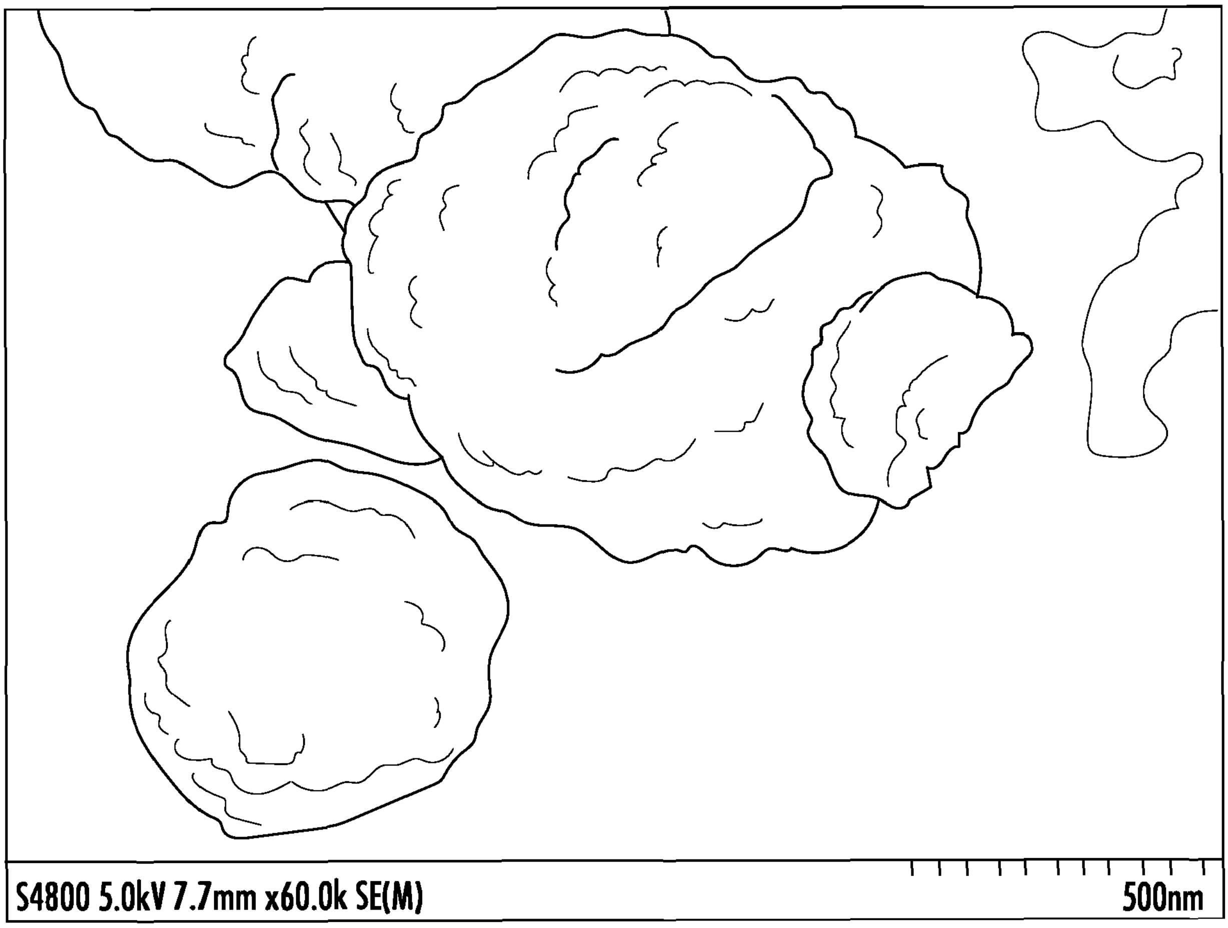


FIG. 2C

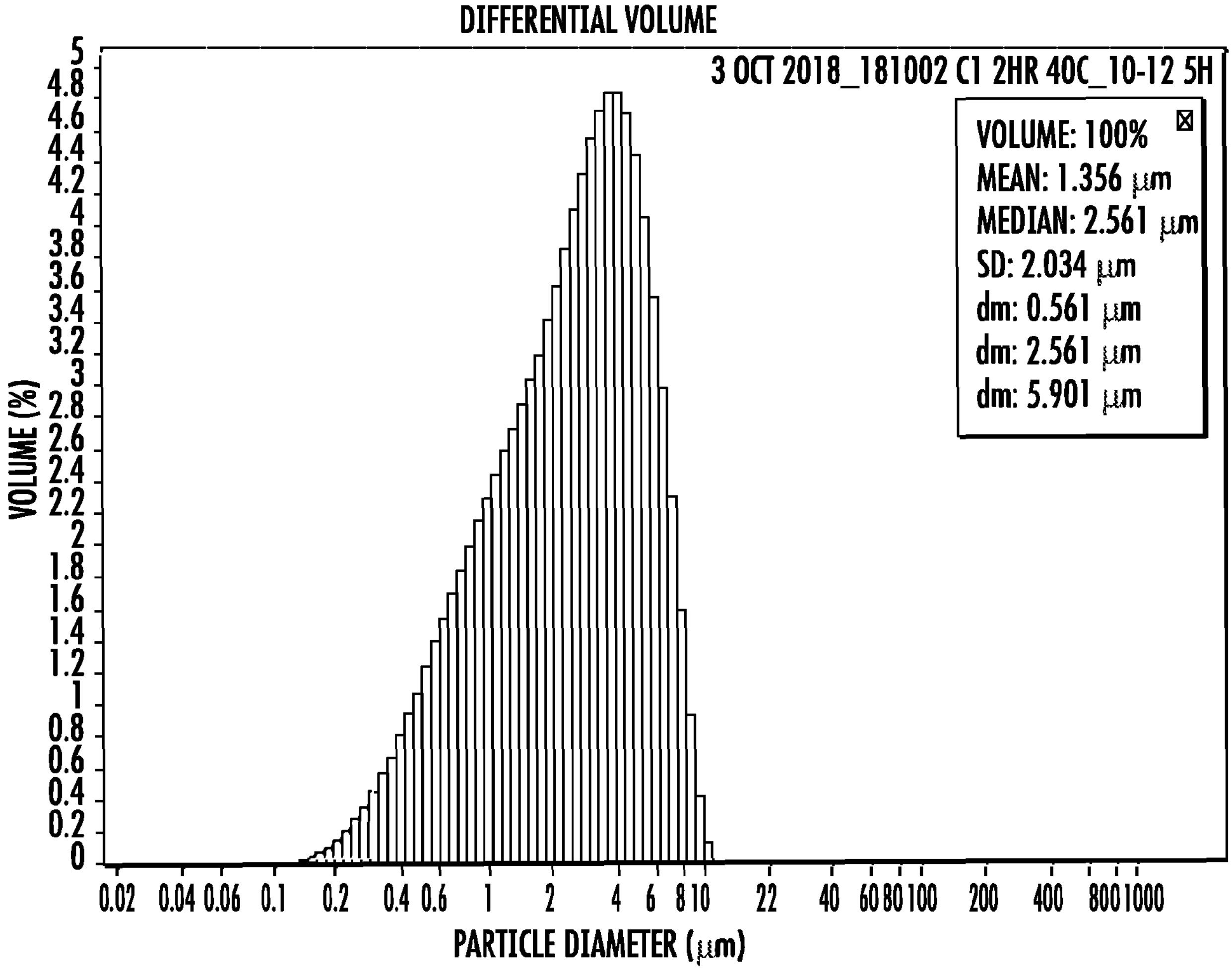
DIFFERENTIAL VOLUME
3 OCT 2018_181002 C1 2HR 40C_10-12 5H
VOLUME: 100%
MEAN: 1.356 µm
MEDIAN: 2.561 µm
SD: 2.034 µm
dm: 0.561 µm
dm: 2.561 µm
dm: 5.901 µm
VOLUME (%)
5
4.8
4.6
4.4
4.2
4
3.8
3.6
3.4
3.2
3
2.8
2.6
2.4
2.2
2
1.8
1.6
1.4
1.2
1
0.8
0.6
0.4
0.2
0
0.02 0.04 0.06 0.1 0.2 0.4 0.6 1 2 4 6 8 10 22 40 60 80 100 200 400 800 1000
PARTICLE DIAMETER (µm)

FIG. 3

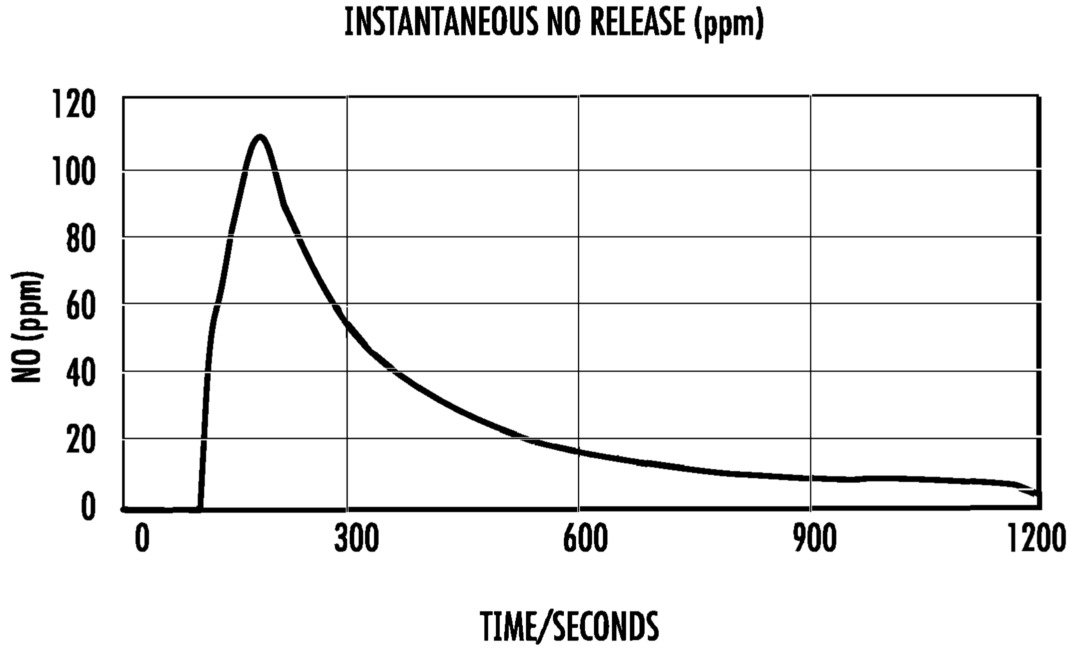
INSTANTANEOUS NO RELEASE (ppm)
NO (ppm)
120
100
80
60
40
20
0
0
300
600
900
1200
TIME/SECONDS

FIG. 4A

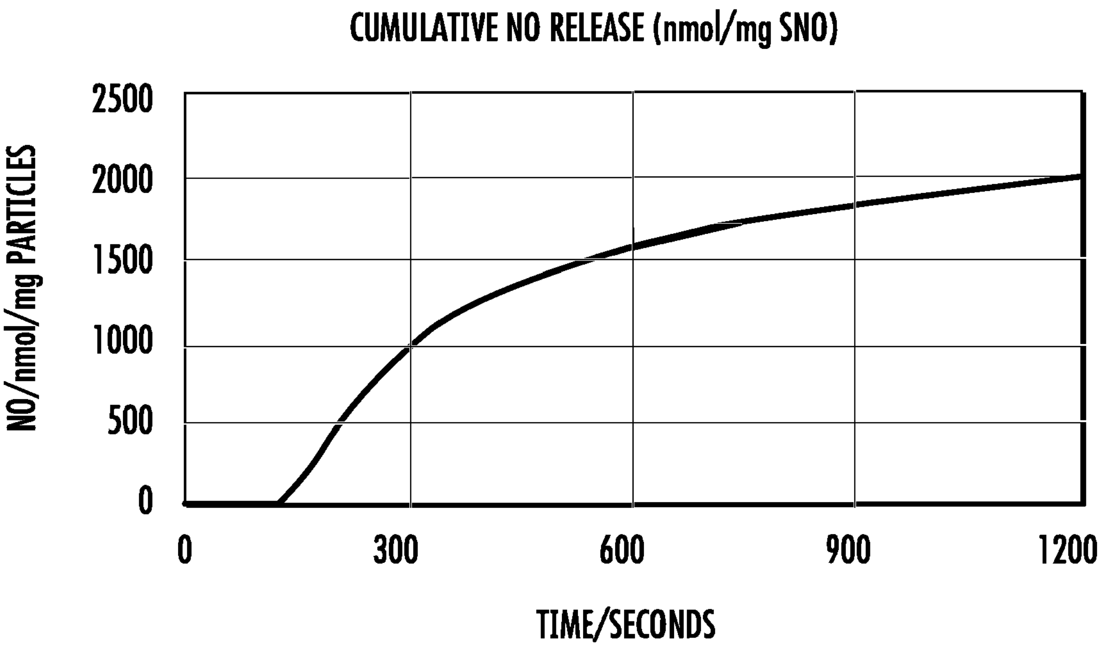
CUMULATIVE NO RELEASE (nmol/mg SNO)
NO/nmol/mg PARTICLES
2500
2000
1500
1000
500
0
0
300
600
900
1200
TIME/SECONDS

FIG. 4B

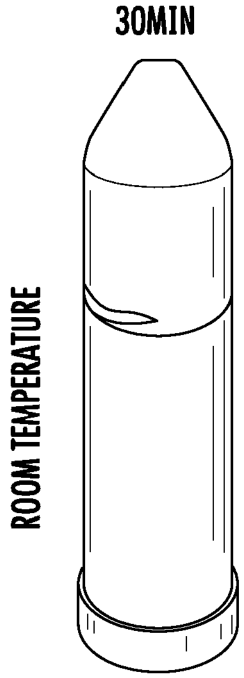


FIG. 5A

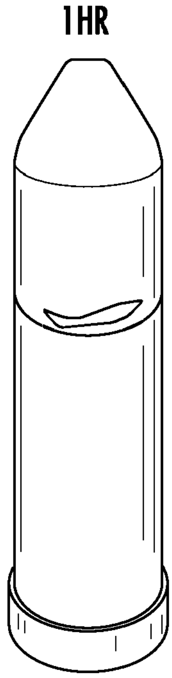


FIG. 5B

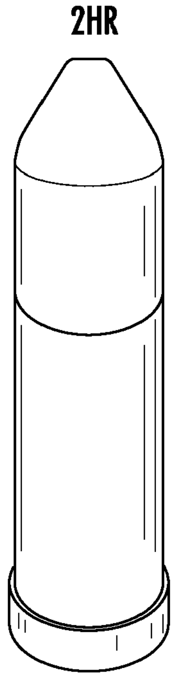


FIG. 5C

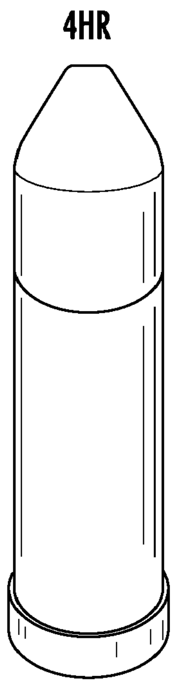


FIG. 5D

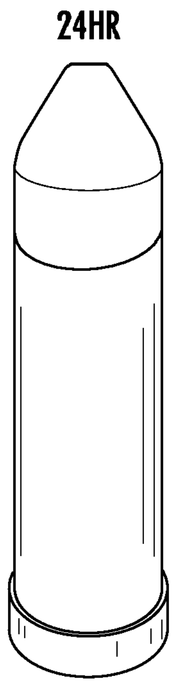


FIG. 5E

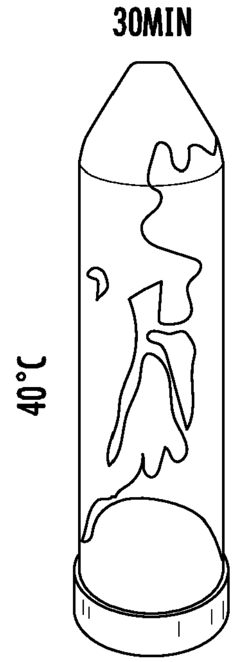


FIG. 5F

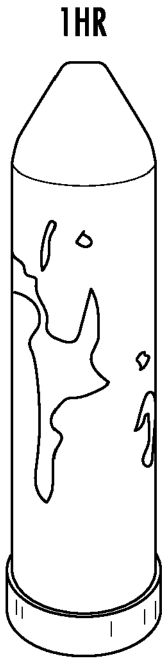


FIG. 5G

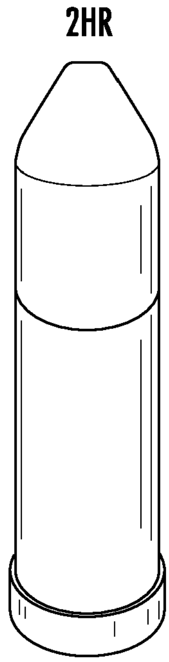


FIG. 5H

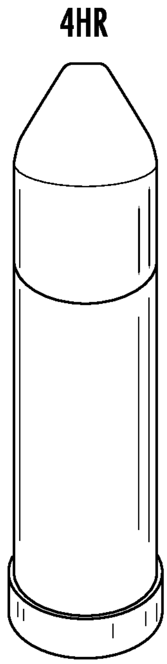


FIG. 5I

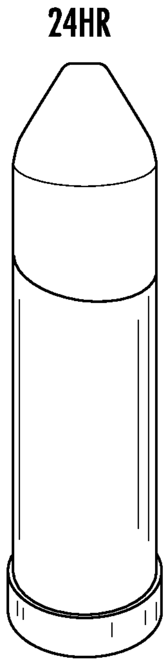


FIG. 5J

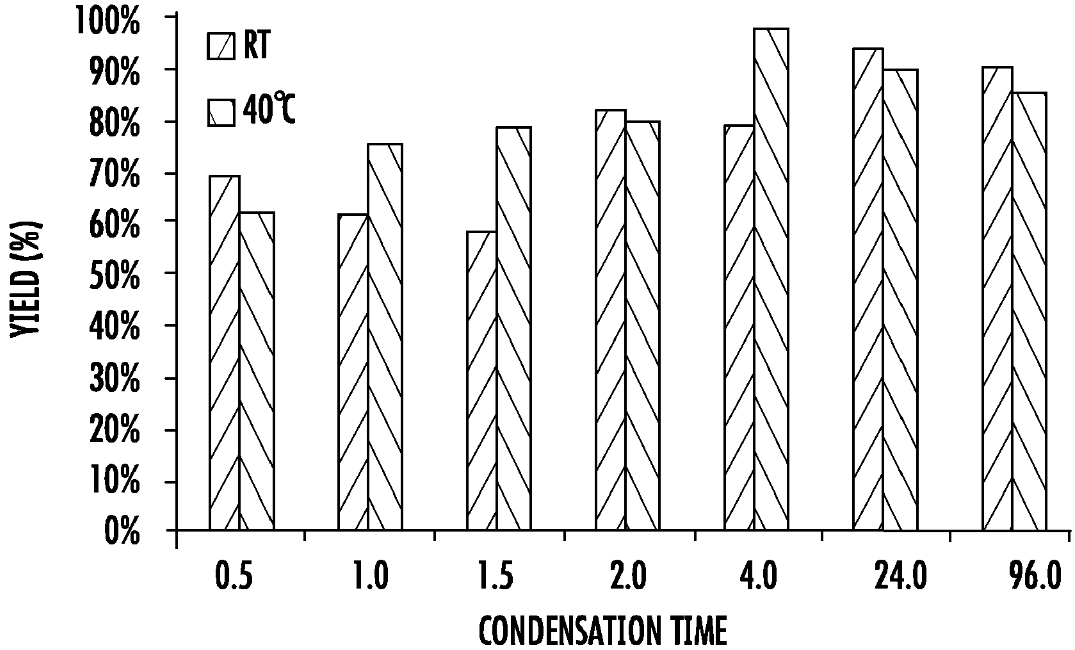
100%
90%
80%
70%
60%
50%
40%
30%
20%
10%
0%
YIELD (%)
RT
40℃
0.5
1.0
1.5
2.0
4.0
24.0
96.0
CONDENSATION TIME

FIG. 6A

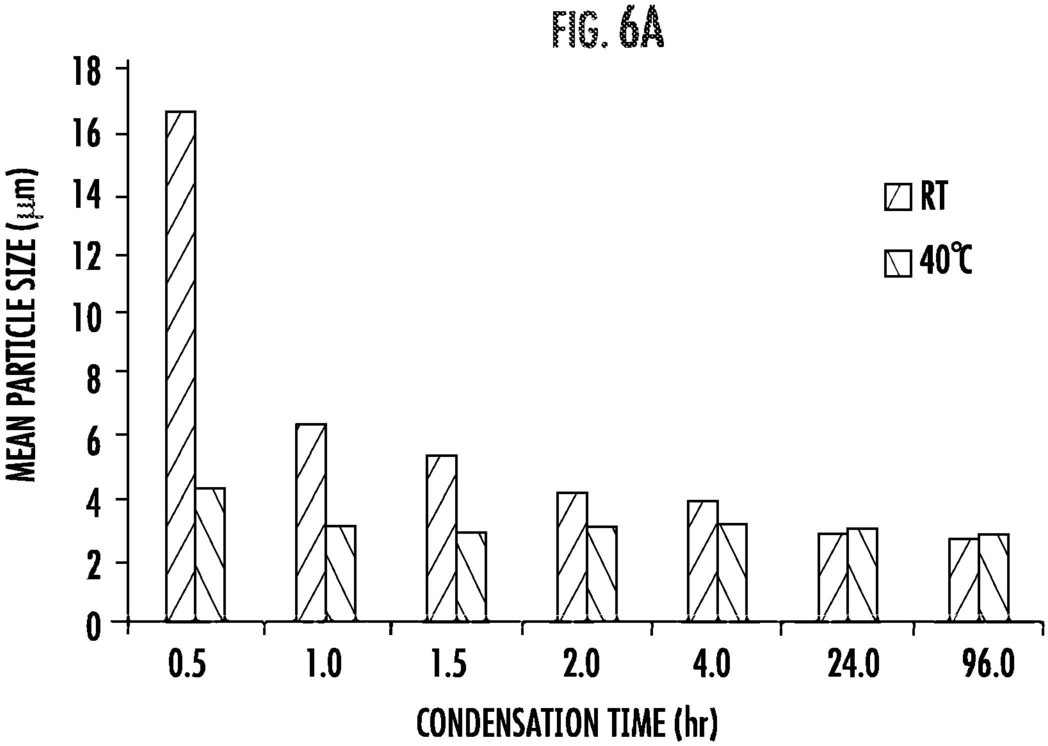
18
16
14
12
10
8
6
4
2
0
MEAN PARTICLE SIZE (μm)
RT
40℃
0.5
1.0
1.5
2.0
4.0
24.0
96.0
CONDENSATION TIME (hr)

FIG. 6B

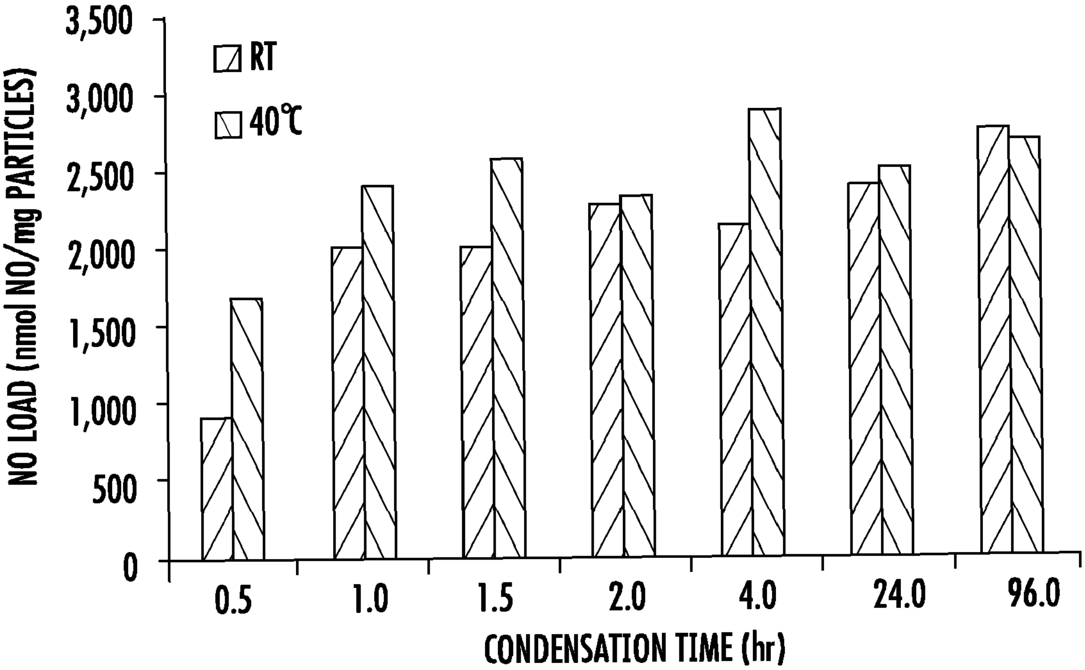
3,500
3,000
2,500
2,000
1,500
1,000
500
0
RT
40℃
NO LOAD (nmol NO/mg PARTICLES)
0.5
1.0
1.5
2.0
4.0
24.0
96.0
CONDENSATION TIME (hr)

FIG. 6C

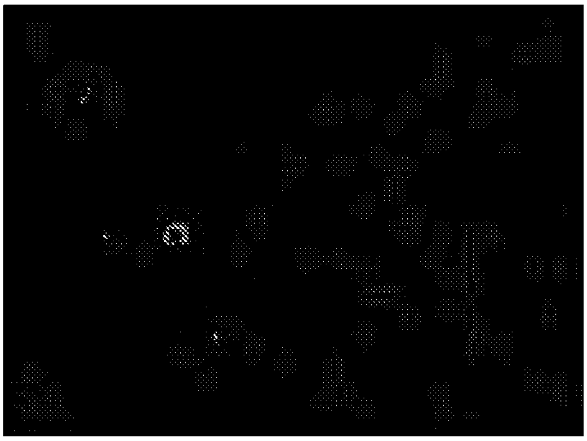

FIG. 7A

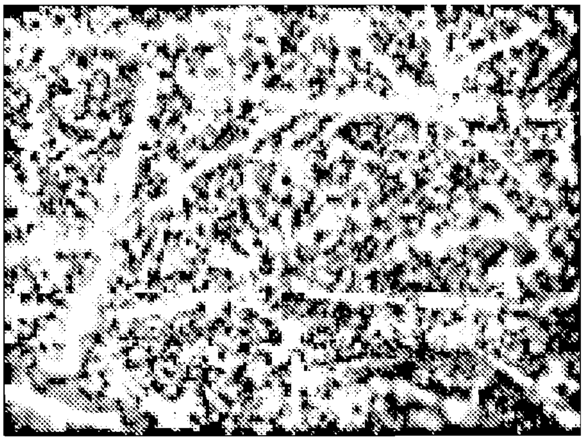

FIG. 7B

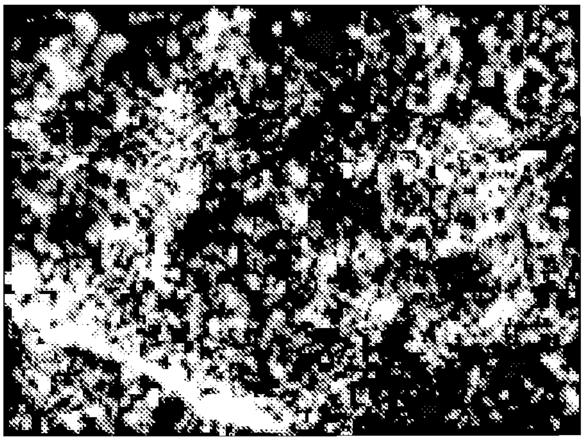

FIG. 7C

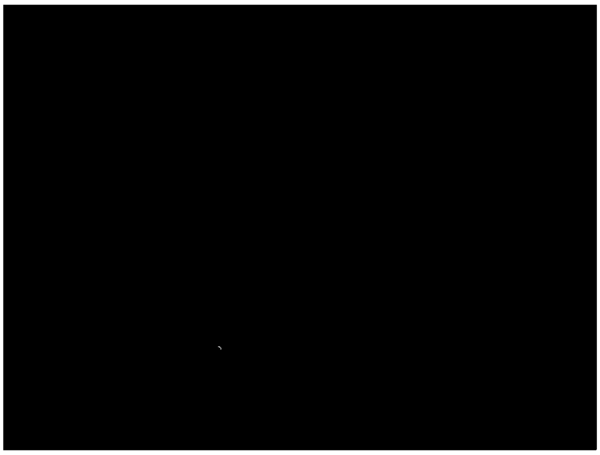

FIG. 7D

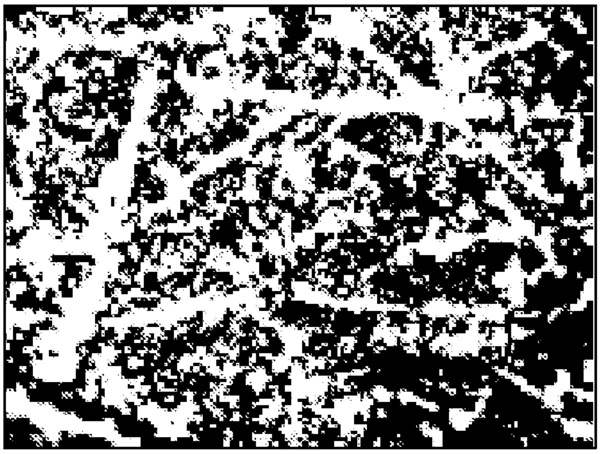

FIG. 7E

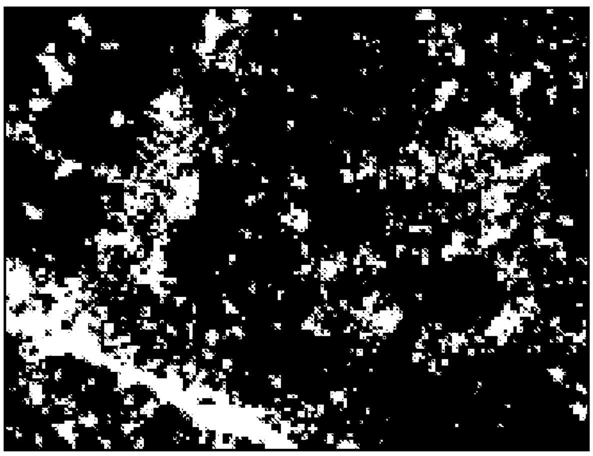

FIG. 7F

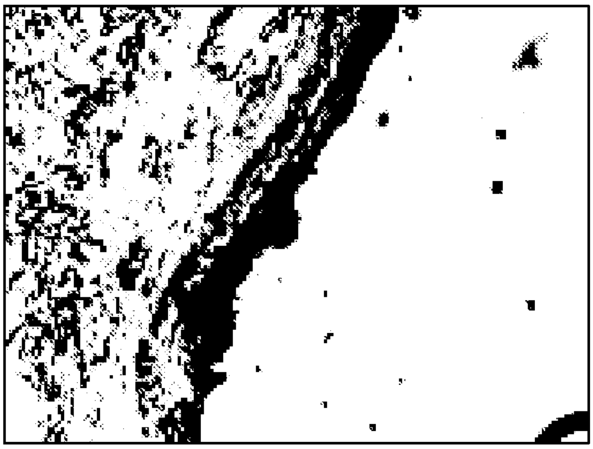

FIG. 8A

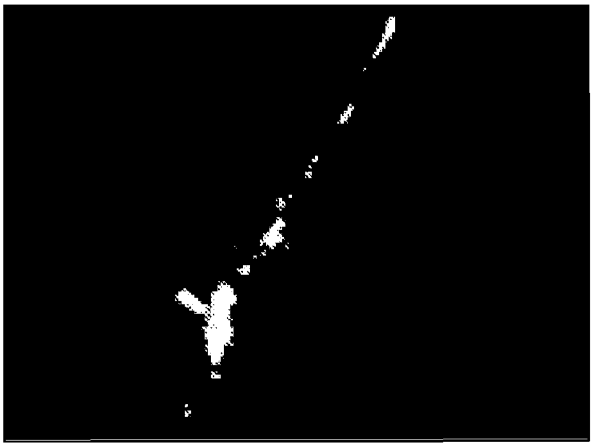

FIG. 8B

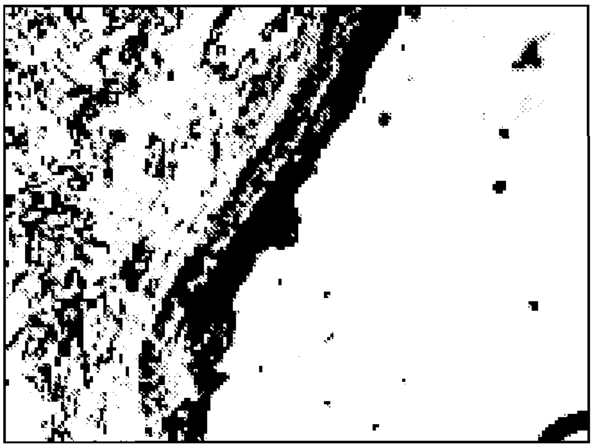

FIG. 8C

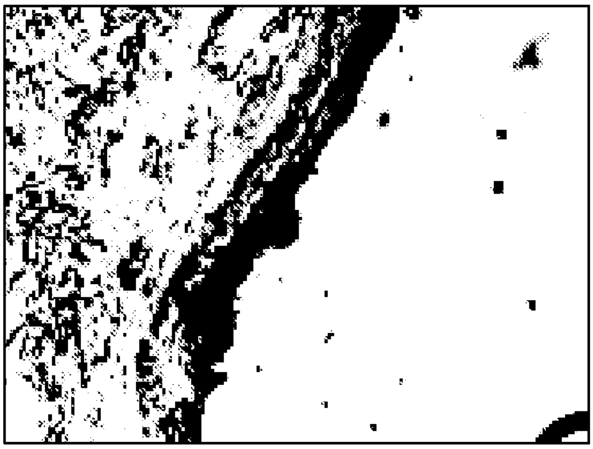

FIG. 8D

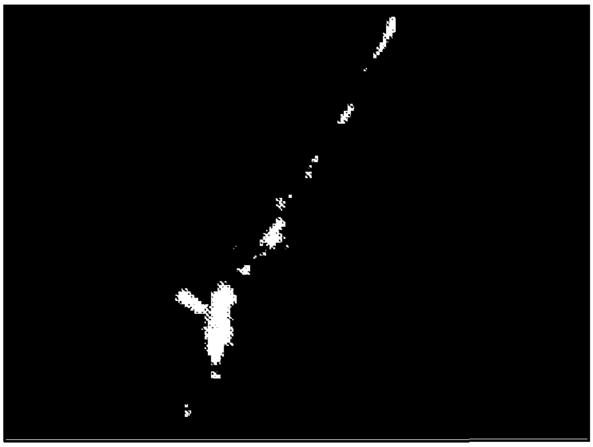

FIG. 8E

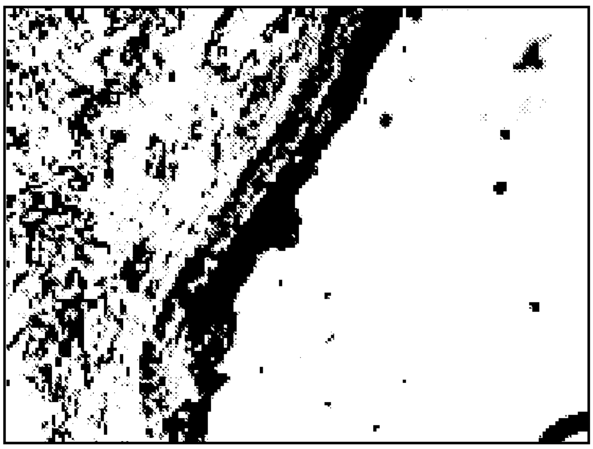

FIG. 8F

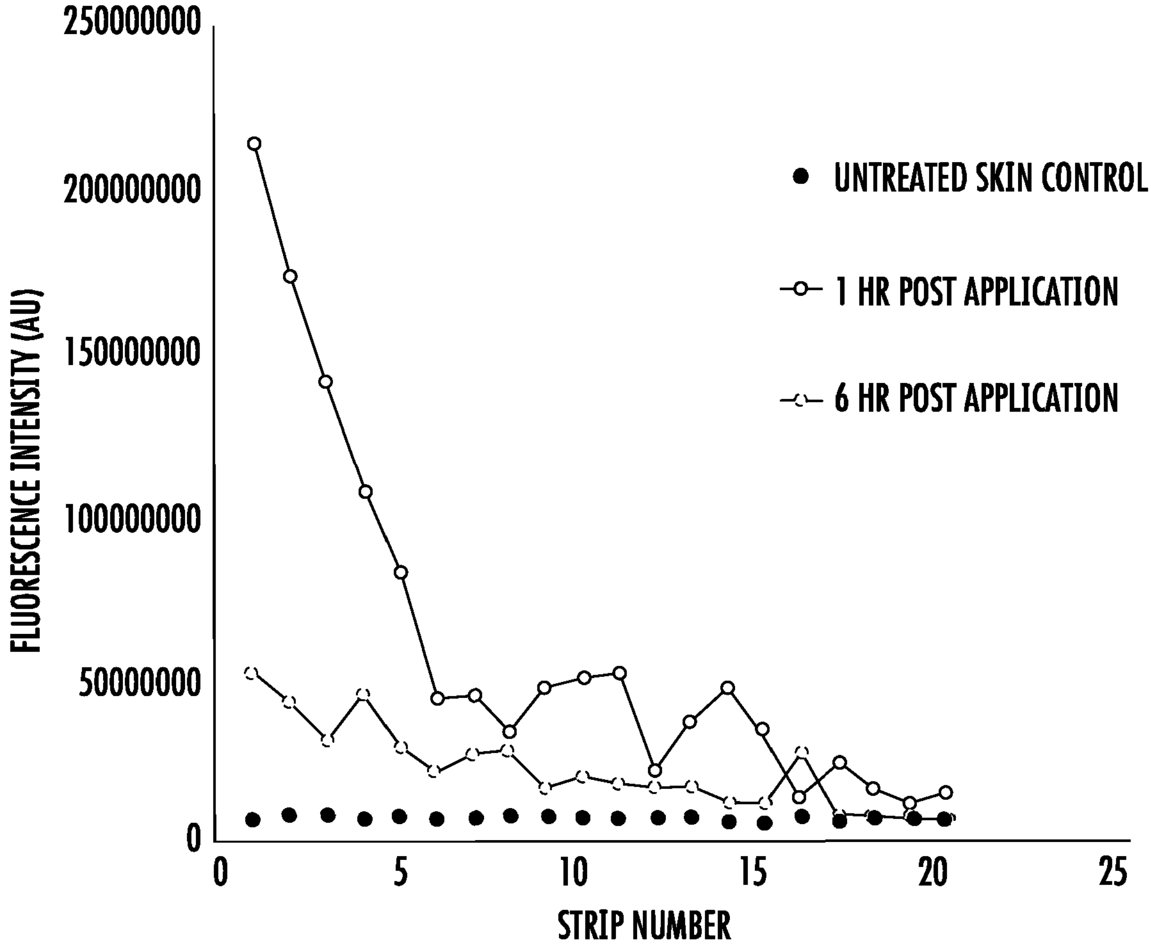
250000000
200000000
150000000
100000000
50000000
0
FLUORESCENCE INTENSITY (AU)
0
5
10
15
20
25
STRIP NUMBER
UNTREATED SKIN CONTROL
1 HR POST APPLICATION
6 HR POST APPLICATION

FIG. 9

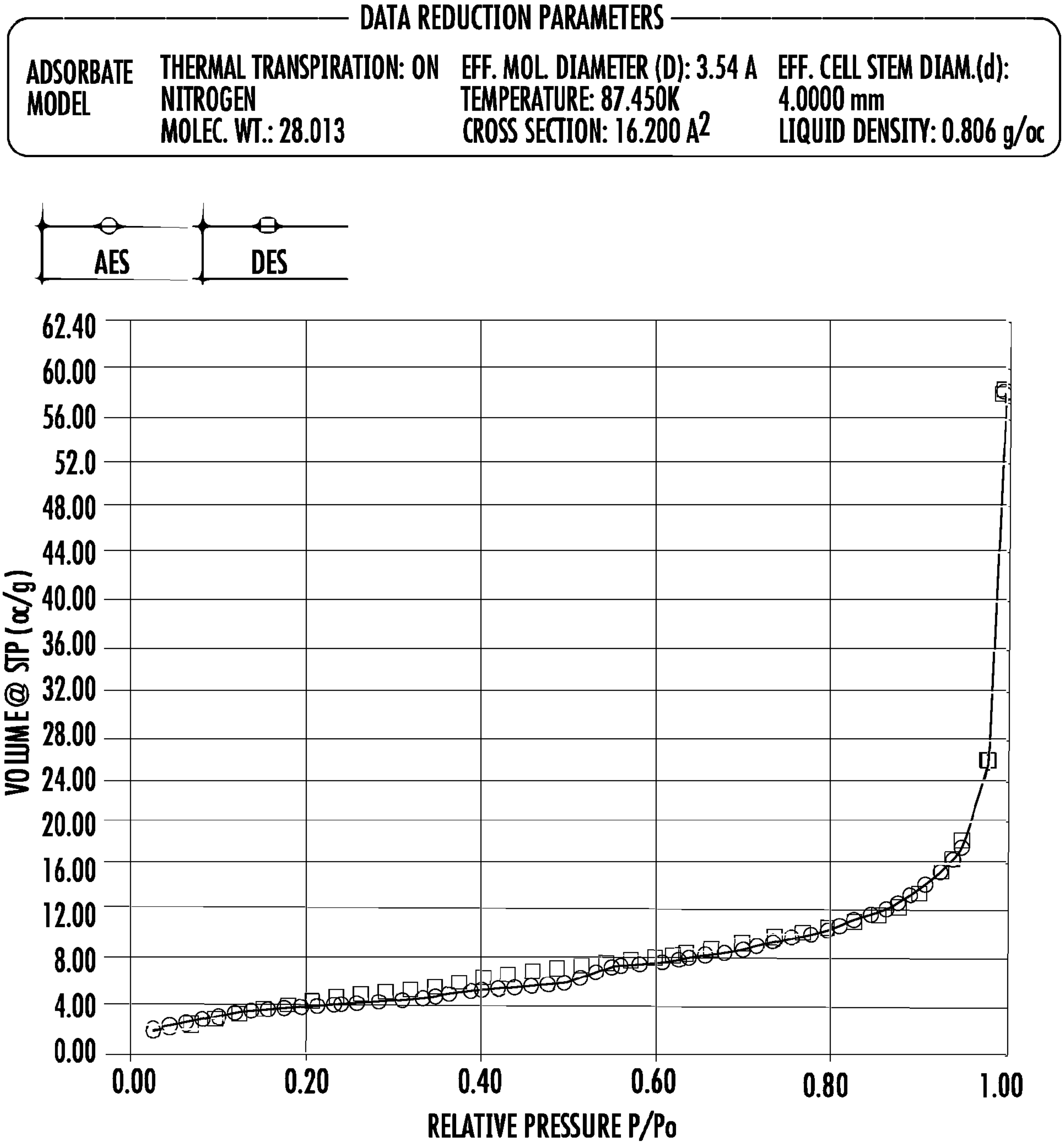
DATA REDUCTION PARAMETERS
ADSORBATE MODEL
THERMAL TRANSPIRATION: ON
NITROGEN
MOLEC. WT.: 28.013
EFF. MOL. DIAMETER (D): 3.54 A
TEMPERATURE: 87.450K
CROSS SECTION: 16.200 A2
EFF. CELL STEM DIAM.(d):
4.0000 mm
LIQUID DENSITY: 0.806 g/oc
AES
DES
62.40
60.00
56.00
52.0
48.00
44.00
40.00
36.00
32.00
28.00
24.00
20.00
16.00
12.00
8.00
4.00
0.00
VOLUME @ STP (oc/g)
0.00
0.20
0.40
0.60
0.80
1.00
RELATIVE PRESSURE P/Po

FIG. 10

### DATA REDUCTION PARAMETERS DATA

| | | | |
|---|---|---|---|
| ADSORBATE MODEL | THERMAL TRANSPIRATION: ON | EFF. MOL. DIAMETER (D): 3.54 A | EFF. CELL STEM DIAM. |
| | NITROGEN | TEMPERATURE 87.450K | (d): 4.0000 mm |
| | MOLEC. WT.: 28.013 | CROSS SECTION: 16.200 $A^2$ | LIQUID DENSITY: 0.806 g/cc |

### MULTI-POINT BET DATA

| RELATIVE PRESSURE [P/Po] | VOLUME @ STP [cc/g] | 1/[W((Po/P)-1)] [1/g] | RELATIVE PRESSURE [P/Po] | VOLUME @ STP [cc/g] | 1/[W((Po/P)-1)] [1/g] |
|---|---|---|---|---|---|
| 1.01057e-01 | 2.9255 | 3.0746e+01 | 2.01013e-01 | 3.8133 | 5.2788e+01 |
| 1.25849e-01 | 3.2057 | 3.5933e+01 | 2.25681e-01 | 4.0671 | 5.7338e+01 |
| 1.50427e-01 | 3.3895 | 4.1797e+01 | 2.50371e-01 | 4.1993 | 6.3637e+01 |
| 1.76724e-01 | 3.6642 | 4.6873e+01 | 2.76215e-01 | 4.3920 | 6.9523e+01 |

### BET SUMMARY

SLOPE = 220.358 1/g

INTERCEPT = 8.310e+00 1/g

CORRELATION COEFFICIENT, r = 0.999619

C CONSTANT = 27.517

SURFACE AREA = 15.230 $m^2/g$

FIG. 11

| MPTS/ TEOS RATIO | MEAN (µm) | MEDIAN (µm) | S.D. (µm) | SPAN (µm) | YIELD (%) | NO LOAD (nmol/mg) | GEL APPEARANCE | GEL DISPERSAL | PARTICLE APPEARANCE |
|---|---|---|---|---|---|---|---|---|---|
| 20/80 | 291.30 | 218.55 | 273.83 | 2.47 | 121.0% | 758 | HARD, TRANSLUCENT | NO | HARD, CRYSTALLINE, TRANSLUCENT, REQUIRED PESTLING |
| 30/70 | 184.84 | 137.22 | 181.23 | 3.15 | 97.0% | 1621 | FIRM, SLIGHTLY TRANSLUCENT | YES, WITH DL ADDED | HARD, SEMI-CRYSTALLINE, WHITE, REQUIRED PESTLING |
| 37/63 | 3.04 | 2.66 | 2.12 | 2.07 | 93.0% | 2780 | NORMAL | YES | FINE WHITE POWDER |
| 40/60 | 4.53 | 4.00 | 3.43 | 2.23 | 80.0% | 2255 | NORMAL | YES | FINE WHITE POWDER |
| 50/50 | 21.07 | 8.63 | 33.46 | 6.18 | 64.0% | 2939 | WEAK GEL | YES | GUMMY, STATICKY, HIGHLY AGGLOMERATED, POOR DISPERSAL |
| 60/40 | 35.26 | 10..47 | 54.17 | 10.44 | 70.0% | 2402 | WEAK GEL | YES | GUMMY, STATICKY, HIGHLY AGGLOMERATED, POOR DISPERSAL |

FIG. 12

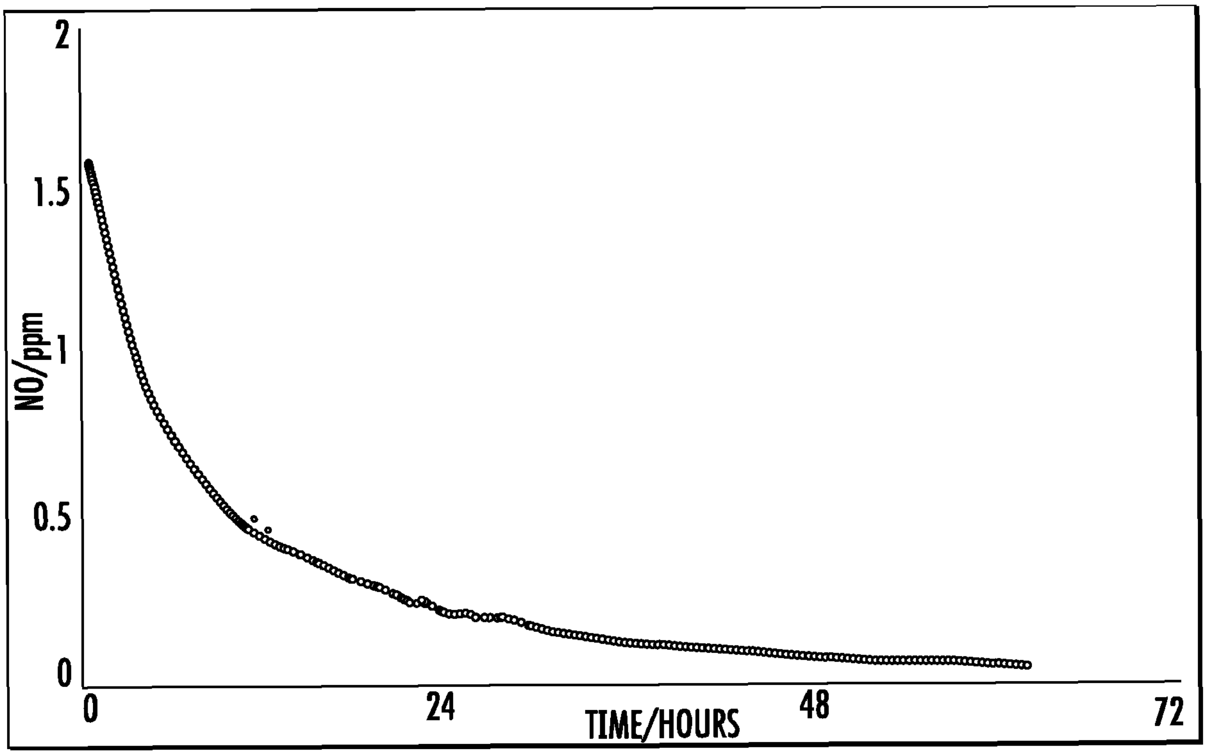


FIG. 13A

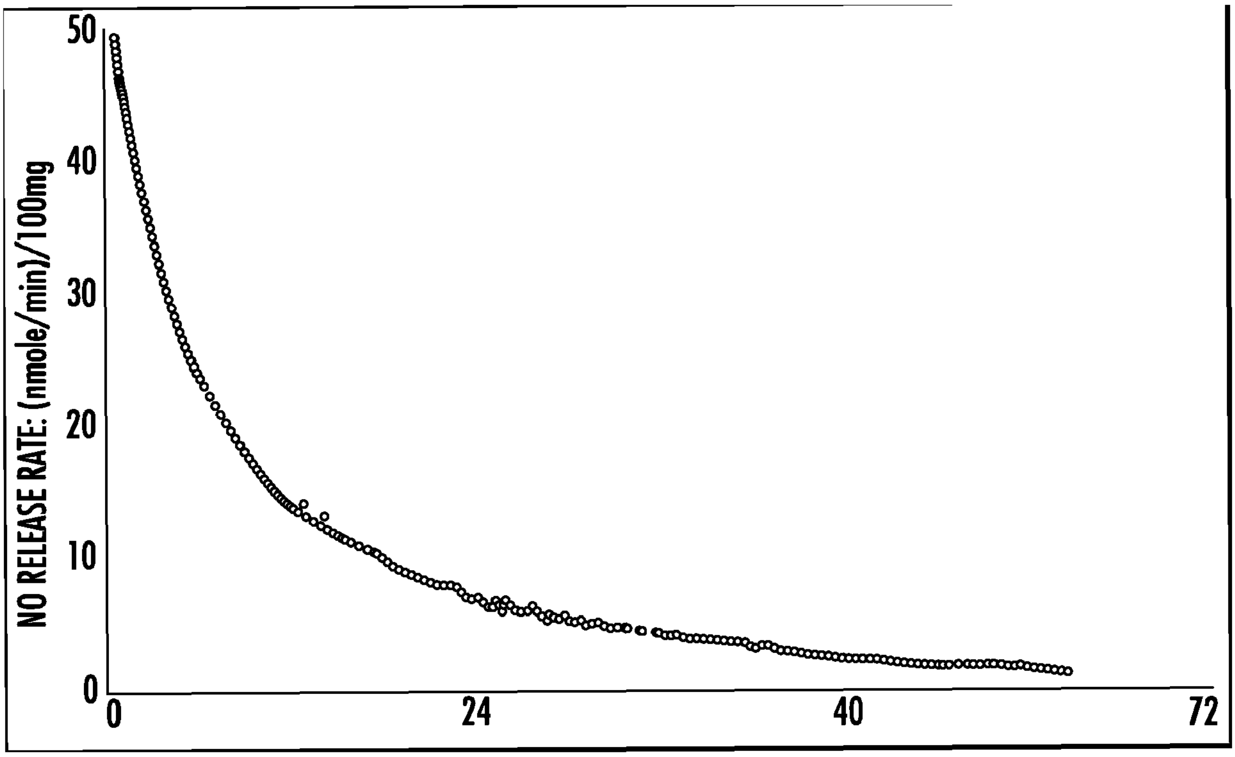


FIG. 13B

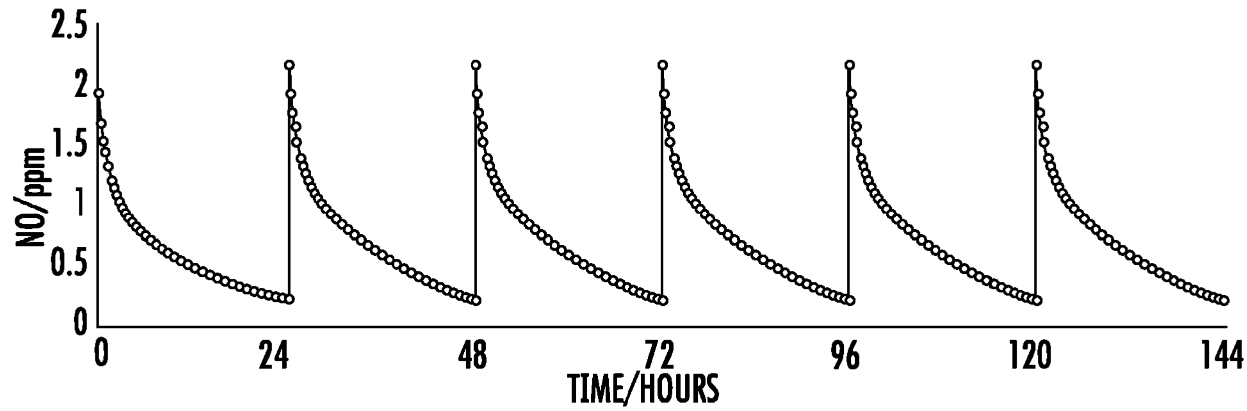


FIG. 14A

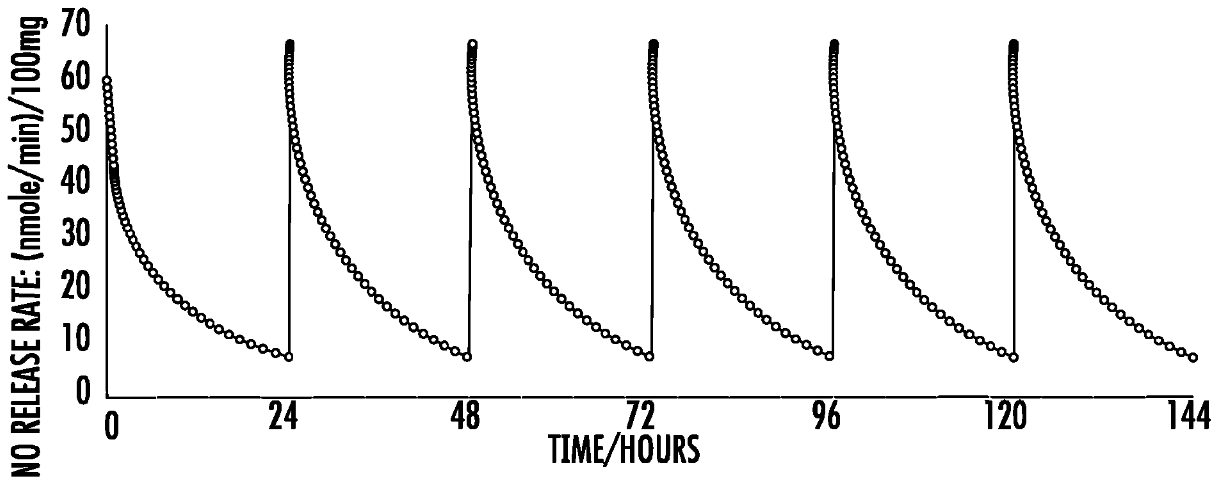


FIG. 14B

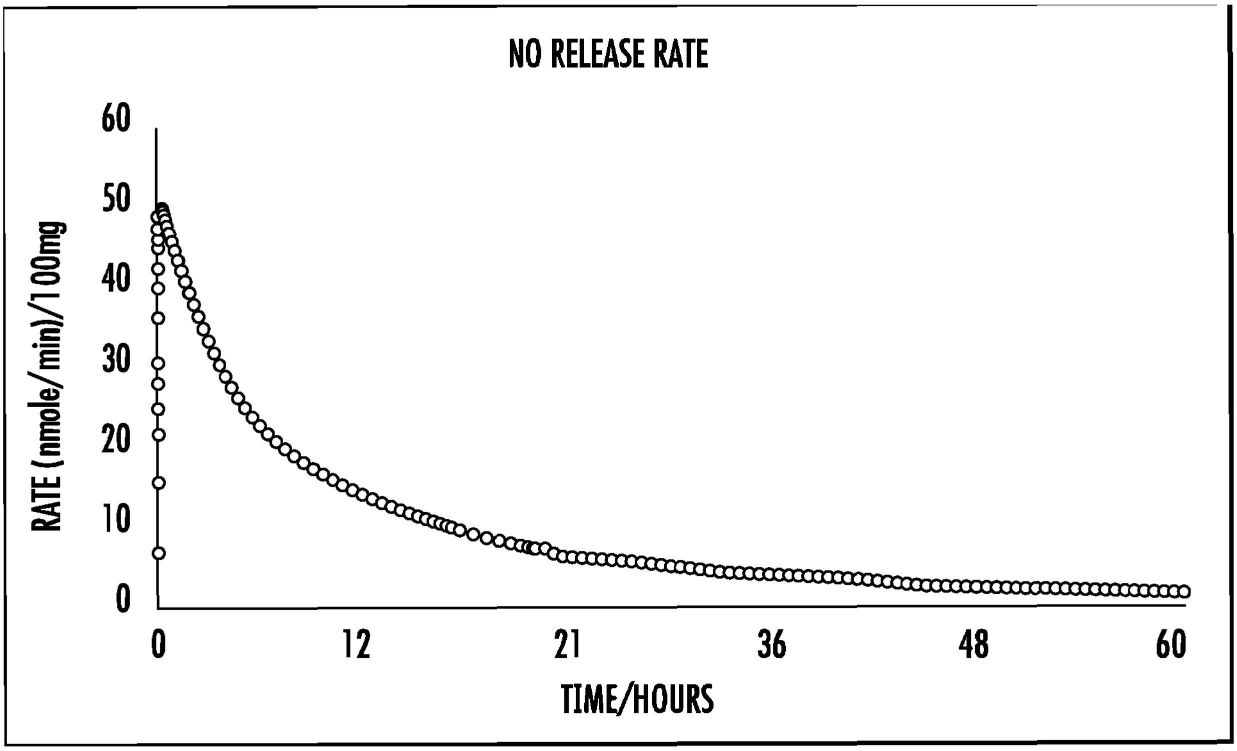
NO RELEASE RATE
RATE (nmole/min)/100mg
60
50
40
30
20
10
0
0
12
21
36
48
60
TIME/HOURS

FIG. 15A

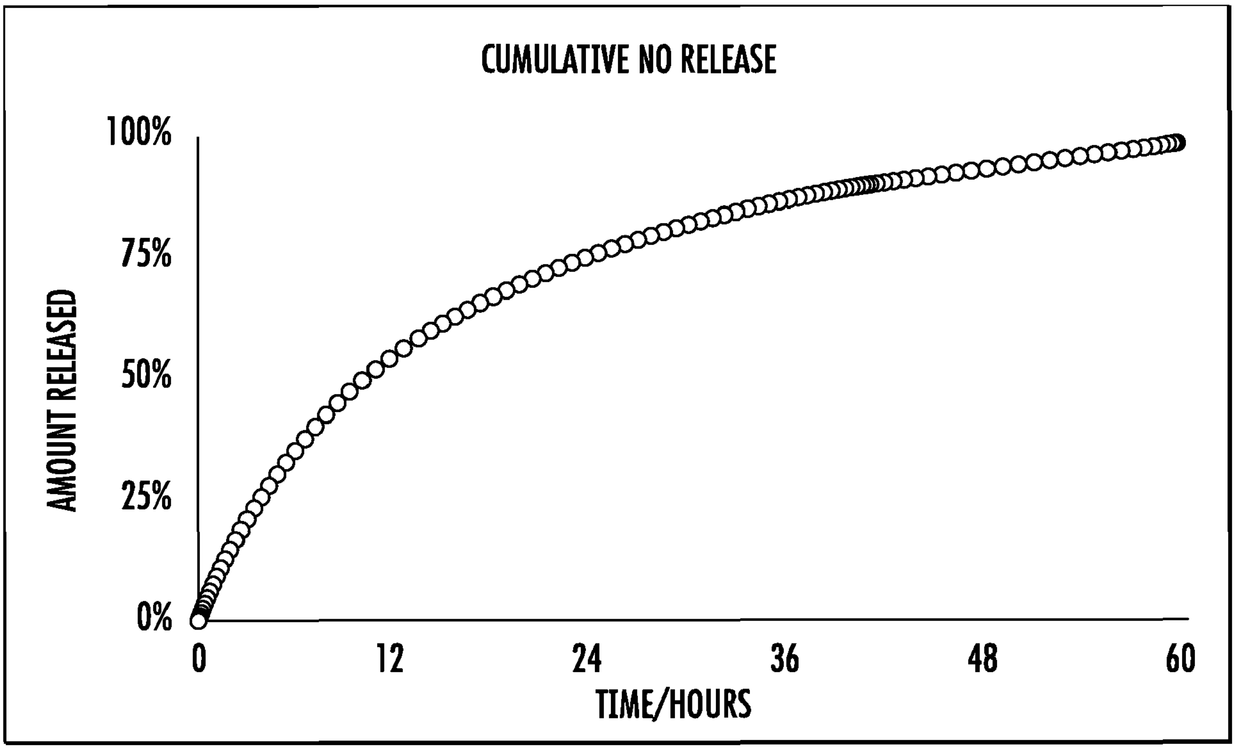
CUMULATIVE NO RELEASE
100%
75%
50%
25%
0%
AMOUNT RELEASED
0
12
24
36
48
60
TIME/HOURS

FIG. 15B

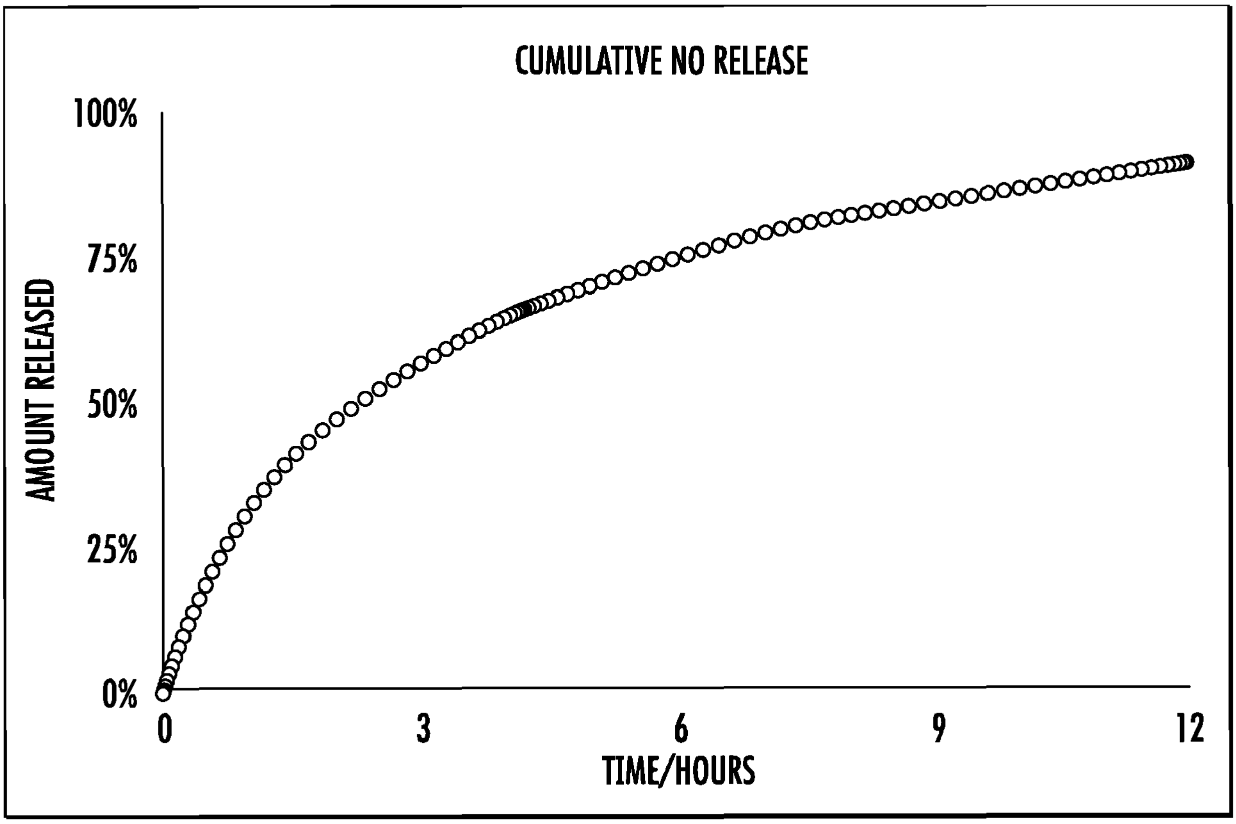
CUMULATIVE NO RELEASE
100%
75%
50%
25%
0%
AMOUNT RELEASED
0
3
6
9
12
TIME/HOURS

FIG. 16

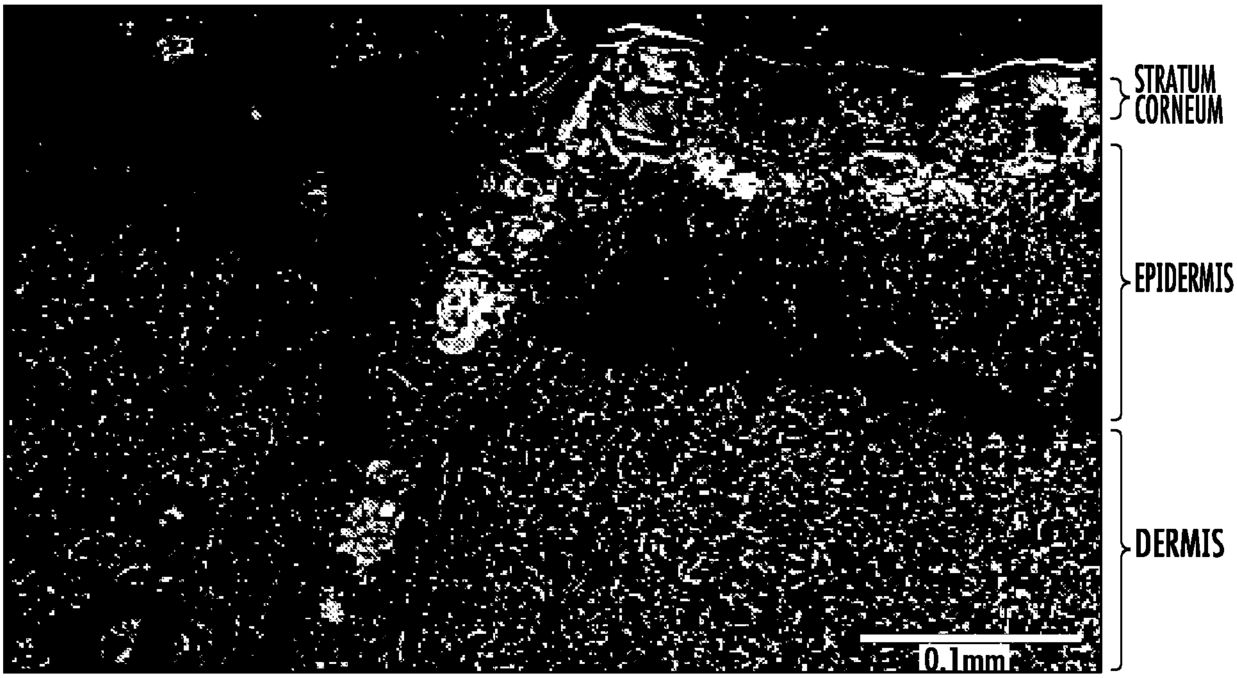


FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62863503